(19) Europäisches Patentamt
European Patent Office
Office européen des brevets

(11) **EP 4 755 324 A1**

(12) **EUROPEAN PATENT APPLICATION**
published in accordance with Art. 153(4) EPC

(43) Date of publication:
**10.06.2026 Bulletin 2026/24**

(51) International Patent Classification (IPC):
**A61B 17/3205** (2006.01)    **A61B 17/32** (2006.01)
**A61B 18/14** (2006.01)

(21) Application number: **24866761.0**

(22) Date of filing: **05.02.2024**

(86) International application number:
**PCT/CN2024/076187**

(87) International publication number:
**WO 2025/060334 (27.03.2025 Gazette 2025/13)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **20.09.2023 CN 202311219288
17.10.2023 CN 202311346415**

(71) Applicant: **Hangzhou AGS MedTech Co., Ltd.
Hangzhou, Zhejiang 311103 (CN)**

(72) Inventors:
• **HAN, Chunqi**
  **Hangzhou, Zhejiang 311106 (CN)**
• **WU, Jiawei**
  **Hangzhou, Zhejiang 311106 (CN)**
• **CHENG, Yonghua**
  **Hangzhou, Zhejiang 311106 (CN)**

(74) Representative: **Wang, Bo
Panovision IP
Ebersberger Straße 3
85570 Markt Schwaben (DE)**

(54) **SNARE**

(57)    Disclosed is a snare device. The snare device includes a snare portion. The snare portion includes a first rigid portion and a second rigid portion. The first rigid portion is connected to the second rigid portion. At least a portion of a structure of the second rigid portion is located at a distal end relative to the first rigid portion. A stiffness of the first rigid portion is less than a stiffness of the second rigid portion. The snare device includes a connection portion. The connection portion is configured to accommodate at least a part of the snare portion. The snare device includes an operation portion. The operation portion is connected to the snare portion. The operation portion is configured to drive a part or all of the snare portion to extend out of or enter the connection portion.

FIG.2

## Description

### CROSS REFERENCE TO RELATED APPLICATIONS

This application claims priority to Chinese Patent Application No. 202311219288.7, filed on September 20, 2023, and Chinese Patent Application No. 202311346415.X, filed on October 17, 2023, the entire contents of which are incorporated herein by reference.

### TECHNICAL FIELD

The present disclosure generally relates to a field of medical devices, and in particular, to a snare device.

### BACKGROUND

A snare device is a commonly used medical instrument in contemporary medicine. The snare device may complete a resection of a lesion by contracting a snare portion. However, during a process of capturing relevant tissues, since an axial change of the snare portion is smaller than a radial change of the snare portion when the snare portion is retracted into a sheath tube, the originally annular snare portion gradually becomes an elongated snare portion, which may lead to that the tissues can't be completely wrapped by the elongated snare portion, resulting in a phenomenon where the snare portion cannot gradually tighten and fix the tissues, thereby affecting a completeness of the lesion resection.

Therefore, it is desirable to provide a snare device that makes morphological changes stable when the snare portion of the snare device is retracted into the sheath tube, avoids a formation of the elongated snare portion, and facilitates the capturing and fixing of the lesion, thereby ensuring the complete resection of the lesion. The snare device may also avoid a phenomenon of easy slippage between the snare device and the lesion, making the contact between them more conforming and more stable.

### SUMMARY

One or more embodiments of the present disclosure provide a snare device, including: a snare portion, including a first rigid portion and a second rigid portion, the first rigid portion being connected to the second rigid portion, at least a part of a structure of the second rigid portion being located at a distal end relative to the first rigid portion, and a stiffness of the first rigid portion being less than a stiffness of the second rigid portion; a connection portion configured to accommodate at least a part of the snare portion; and an operation portion connected to the snare portion, the operation portion driving a part or all of the snare portion to extend out of or enter the connection portion.

In some embodiments, when the snare portion does not enter the connection portion or partially enters the connection portion, a range of a diameter-length ratio of the snare portion is 0.5-1. The diameter-length ratio is a ratio of a dimension of a snaring part of the snare portion along a radial direction of the snare device to a dimension of the snaring part along an axial direction of the snare device.

In some embodiments, during a process of the snare portion partially entering the connection portion, a first change rate of a snaring part of the snare portion is not greater than a second change rate of the snaring part. The first change rate refers to a ratio of a change of a dimension of the snaring part along a radial direction of the snare device to an initial radial dimension; and the second change rate refers to a ratio of a change of a dimension of the snaring part along an axial direction of the snare device to an initial axial dimension.

In some embodiments, the first rigid portion includes a first connection segment, and the second rigid portion includes a second connection segment. The second connection segment is located at a distal end relative to the first connection segment, and a stiffness of the first connection segment is less than a stiffness of the second connection segment.

In some embodiments, two ports at a distal end of the first connection segment are respectively connected to two ports at a proximal end of the second connection segment; and two ports at a proximal end of the first connection segment are connected to the operation portion.

In some embodiments, when the snare portion does not enter the connection portion, a ratio range of a dimension of the second connection segment along the axial direction of the snare device to a dimension of the snare portion along the axial direction of the snare device is 0.1-0.35.

In some embodiments, a ratio range of the stiffness of the first connection segment to the stiffness of the second connection segment is 0.2-0.9.

In some embodiments, both the first connection segment and the second connection segment include an arc-shaped structure, a curvature of an arc-shaped structure of the first connection segment is less than a curvature of an arc-shaped structure of the second connection segment.

In some embodiments, a ratio range of the curvature of the arc-shaped structure of the first connection segment to the curvature of the arc-shaped structure of the second connection segment is 0.1-0.85.

In some embodiments, a hardness of the second connection segment is greater than a hardness of the first connection segment.

In some embodiments, a first feature dimension is less than a second feature dimension. The first feature dimension is a feature dimension of a cross-section of the first connection segment perpendicular to an axial direction of the first connection segment; and the second feature dimension is a feature dimension of a cross-section of the second connection segment perpendicular to an axial direction of the second connection segment.

In some embodiments, a ratio range of the first feature dimension to the second feature dimension is 0.5-0.95.

In some embodiments, the first connection segment includes at least two first connection units, and the second connection segment includes at least one second connection unit; and a first count of strands is greater than a second count of strands. The first count of strands is a count of first connection units of the first connection segment, and the second count of strands is a count of a second connection unit of the second connection segment.

In some embodiments, when an absolute value of a difference between a first feature dimension and a second feature dimension is less than or equal to a first preset threshold, the first count of strands is greater than the second count of strands. The first feature dimension is a feature dimension of a cross-section of the first connection segment perpendicular to an axial direction of the first connection segment; and the second feature dimension is a feature dimension of a cross-section of the second connection segment perpendicular to an axial direction of the second connection segment.

In some embodiments, the first preset threshold is any value between 0 mm and 0.1 mm.

In some embodiments, a shape of a second cross-section is a circle, a square, a hexagon, or a combination thereof, and a shape of a first cross-section is a semicircle, a triangle, a rectangle, or a combination thereof. The first cross-section is a cross-section of the first connection segment perpendicular to an axial direction of the first connection segment; and the second cross-section is a cross-section of the second connection segment perpendicular to an axial direction of the second connection segment.

In some embodiments, the second connection segment includes a base connection segment and a stiffness reinforcement member, the stiffness reinforcement member being fixed on the base connection segment.

In some embodiments, the first connection segment and the base connection segment are an integral structure.

In some embodiments, the second rigid portion further includes a third connection segment. The third connection segment is located at a proximal end relative to the first connection segment; the stiffness of the first connection segment is less than the stiffness of the second connection segment; and the stiffness of the first connection segment is less than a stiffness of the third connection segment.

In some embodiments, the two ports at the distal end of the first connection segment are respectively connected to the two ports at the proximal end of the second connection segment; the two ports at the proximal end of the first connection segment are respectively connected to two ports at a distal end of the third connection segment; and two ports at a proximal end of the third connection segment are connected to the operation portion.

In some embodiments, when the snare portion does not enter the connection portion, a ratio range of a dimension of the third connection segment along the axial direction of the snare device to the dimension of the snare portion along the axial direction of the snare device is 1/10-2/5.

In some embodiments, the stiffness of the third connection segment is the same as the stiffness of the second connection segment; or the stiffness of the third connection segment is greater than the stiffness of the second connection segment; or the stiffness of the third connection segment is less than the stiffness of the second connection segment.

In some embodiments, when the stiffness of the third connection segment is the same as the stiffness of the second connection segment, a ratio range of the stiffness of the first connection segment to the stiffness of the second connection segment is 0.2-0.9; or when the stiffness of the third connection segment is greater than the stiffness of the second connection segment, a ratio range of the stiffness of the second connection segment, the stiffness of the first connection segment, and the stiffness of the third connection segment is (1.1-3.4):1:(1.4-3.6); or when the stiffness of the third connection segment is less than the stiffness of the second connection segment, a ratio range of the stiffness of the second connection segment, the stiffness of the first connection segment, and the stiffness of the third connection segment is (1.1-3.5):1:(1-3.2).

In some embodiments, a hardness of the third connection segment is greater than the hardness of the first connection segment.

In some embodiments, the first feature dimension is less than a third feature dimension. The first feature dimension is a feature dimension of a cross-section of the first connection segment perpendicular to the axial direction of the first connection segment; and the third feature dimension is a feature dimension of a cross-section of the third connection segment perpendicular to an axial direction of the third connection segment.

In some embodiments, a ratio range of the first feature dimension to the third feature dimension is 0.5-0.95.

In some embodiments, the first connection segment includes the at least two first connection units, the third connection segment includes at least one third connection unit; and the first count of strands is greater than a third count of strands. The first count of strands is the count of the first connection units of the first connection segment, and the third count of strands is a count of a third connection unit of the third connection segment.

In some embodiments, when an absolute value of a difference between the first feature dimension and a third feature dimension is less than or equal to a second preset threshold, the first count of strands is greater than the third count of strands. The first feature dimension is a feature dimension of a cross-section of the first connection segment perpendicular to the axial direction of the first connection segment; and the third feature dimension is a feature dimension of a cross-section of the third connection segment perpendicular to an axial direction of the third connection segment.

In some embodiments, the second preset threshold is any value between 0 mm and 0.1 mm.

In some embodiments, a shape of a third cross-section is a circle, a square, a hexagon, or a combination thereof, and a shape of the first cross-section is a semicircle, a triangle, a rectangle, or a combination thereof. The first cross-section is a cross-section of the first connection segment perpendicular to the axial direction of the first connection segment; and the third cross-section is a cross-section of the third connection segment perpendicular to an axial direction of the third connection segment.

In some embodiments, the third connection segment includes a base connection segment and a stiffness reinforcement member, the stiffness reinforcement member being fixed on the base connection segment.

In some embodiments, the snare portion is a symmetrical structure with an axis along an axial direction of the snare device as a symmetry axis; and/or the second rigid portion is a symmetrical structure with an axis along the axial direction of the snare device as a symmetry axis.

In some embodiments, the connection portion includes a first traction member and a sheath tube. The first traction member is disposed in the sheath tube; the first traction member connects the snare portion and the operation portion; and the operation portion drives a part or all of the snare portion to extend out of or enter the sheath tube through the first traction member.

In some embodiments, at least a part of one of the first rigid portion and the second rigid portion that is closer to the first traction member is an integral structure with the first traction member; and/or the at least a part of one of the first rigid portion and the second rigid portion that is closer to the first traction member is fixedly connected to the first traction member.

In some embodiments, the snare portion includes a base portion and an adjustment portion. The adjustment portion is slidably connected to the base portion; two ports at a proximal end of the base portion are connected to a distal end of the first traction member; the adjustment portion includes the first rigid portion and the second rigid portion; and the operation portion drives a part or all of the base portion and a part or all of the adjustment portion to extend out of or enter the sheath tube.

In some embodiments, the connection portion further includes a second traction member, the second traction member being disposed in the sheath tube. The second traction member and the first traction member are both configured to be movable along an axial direction of the sheath tube, and the second traction member is slidable relative to the first traction member. The second traction member includes a first pulling cable and a second pulling cable, a port at a distal end of the first pulling cable being connected to a port at a proximal end of the adjustment portion, and a port at a distal end of the second pulling cable being connected to another port at a proximal end of the adjustment portion; and a sliding movement of the first pulling cable and the second pulling cable along the axial direction of the sheath tube drives the adjustment portion to slide relative to the base portion.

In some embodiments, the operation portion includes an adjustment member. The adjustment member is connected to two ports at a proximal end of the snare portion through the first traction member, and the adjustment member drives the two ports at the proximal end of the snare portion to move synchronously or asynchronously.

In some embodiments, the first rigid portion and/or the second rigid portion are configured with a marker.

## BRIEF DESCRIPTION OF THE DRAWINGS

The present disclosure will be further illustrated by way of exemplary embodiments, which will be described in detail by means of the accompanying drawings. These embodiments are not limiting, and in these embodiments, the same counting denotes the same structure, wherein:

FIG. 1 is a module diagram illustrating an exemplary snare device according to some embodiments of the present disclosure;
FIG. 2 is a schematic diagram illustrating a snare device according to some embodiments of the present disclosure;
FIG. 3 is a schematic diagram illustrating a snare portion according to some embodiments of the present disclosure;
FIG. 4 is a schematic diagram illustrating another snare device according to some embodiments of the present disclosure;
FIG. 5 is a schematic diagram illustrating another snare portion according to some embodiments of the present disclosure;

FIG. 6A is a schematic diagram illustrating a use of a snare device;

FIG. 6B is a schematic diagram illustrating a use of a snare device according to some embodiments of the present disclosure;

FIG. 7 is a schematic diagram illustrating changes during operation of a snare portion according to some embodiments of the present disclosure;

FIG. 8A is a schematic diagram illustrating an oval snare portion according to some embodiments of the present disclosure;

FIG. 8B is a schematic diagram illustrating a circular snare portion according to some embodiments of the present disclosure;

FIG. 8C is a schematic diagram illustrating a heart-shaped snare portion according to some embodiments of the present disclosure;

FIG. 8D is a schematic diagram illustrating a hexagonal snare portion according to some embodiments of the present disclosure;

FIG. 8E is a schematic diagram illustrating a shield-shaped snare portion according to some embodiments of the present disclosure;

FIG. 8F is a schematic diagram illustrating a polygonal snare portion according to some embodiments of the present disclosure;

FIG. 9 is a schematic diagram illustrating a first connection unit in a first connection segment according to some embodiments of the present disclosure;

FIG. 10 is a schematic diagram illustrating another snare portion according to some embodiments of the present disclosure;

FIG. 11 is a schematic diagram illustrating another snare portion according to some embodiments of the present disclosure;

FIG. 12A is a schematic diagram illustrating an enlarged portion of a snare portion according to some embodiments of the present disclosure;

FIG. 12B is another schematic diagram illustrating an enlarged portion of a snare portion according to some embodiments of the present disclosure;

FIG. 13 is another schematic diagram illustrating an enlarged cross-section of a part of a snare portion according to some embodiments of the present disclosure;

FIG. 14 is a schematic diagram illustrating a structure of a part of a first traction member and a second traction member according to some embodiments of the present disclosure;

FIG. 15 is a schematic diagram illustrating a part of an operation portion according to some embodiments of the present disclosure; and

FIG. 16 is a schematic diagram illustrating a method for testing a material stiffness according to some embodiments of the present disclosure.

Reference numerals: 100, snare device; 110, operation portion; 111, handle; 112, first slide member; 113, second slide member; 120, connection portion; 121, sheath tube; 1211, outer sheath tube; 1212, inner sheath tube; 122, first traction member; 123, connection tube; 124, support tube; 125, connection member; 126, second traction member; 126-1, first pulling cable; 126-2, second pulling cable; 127, limit ring; 130, snare portion; 131, first connection segment; 131-1, sub-connection segment; 131-a, first connection unit; 132, second connection segment; 132-1, base connection segment; 132-2, stiffness reinforcement member; 133, third connection segment; 134, guide structure; 135, connection ring; 136, first adjustment channel; 137, second adjustment channel; 1301, base portion; 1302, adjustment portion; 200, lesion; 310, material; 320, deformed material; 330, support member; 340, force application member.

## DETAILED DESCRIPTION

To more clearly illustrate the technical solutions in the embodiments of the present disclosure, the accompanying drawings used in the description of the embodiments will be briefly introduced below. Obviously, the accompanying drawings in the following description are merely some examples or embodiments of the present disclosure. For those skilled in the art, the present disclosure may be applied to other similar scenarios based on these drawings without creative efforts. Unless obviously obtained from the context or the context illustrates otherwise, the same numeral in the drawings refers to the same structure or operation.

It should be understood that the terms "system," "device," "unit," and/or "module" used herein are manners for distinguishing components, elements, parts, sections, or assemblies of different levels. However, if other words can achieve the same purpose, the words may be replaced by other expressions.

As shown in the present disclosure and the claims, unless the context clearly indicates an exception, the words "a," "an," "one," and/or "the" are not specifically singular and may also include the plural. Generally speaking, the terms "include" and "comprise" only indicate the inclusion of explicitly identified steps and elements, and these steps and elements do not constitute an exclusive list. A method or device may also include other steps or elements.

The present disclosure uses flowcharts to illustrate the steps performed by the system according to the embodiments of the present disclosure. It should be understood that the preceding or following operations are not necessarily performed precisely in sequence. On the contrary, the steps may be processed in reverse order or simultaneously. At the same time, other steps may be added to these processes, or one or several steps may be removed from these processes.

Under a structural design of certain snare devices, during the operation of the snare device, as the snare tightens, the snaring part becomes slender. The snaring part is a part of a snare portion located outside a connection portion (e.g., a sheath tube in a connection portion). Merely by way of example, dimensional changes of the snaring part are shown in the following table.

| Stage | Radial Dimension (mm) | Axial Dimension (mm) | Diameter-Length Ratio |
| --- | --- | --- | --- |
| Initial Stage | 27 | 42 | 0.64 |
| First Stage | 20 | 41 | 0.49 |
| Second Stage | 15 | 38 | 0.39 |
| Third Stage | 10 | 28 | 0.36 |

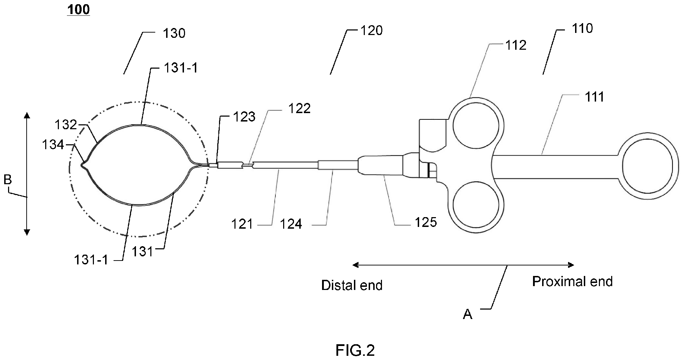

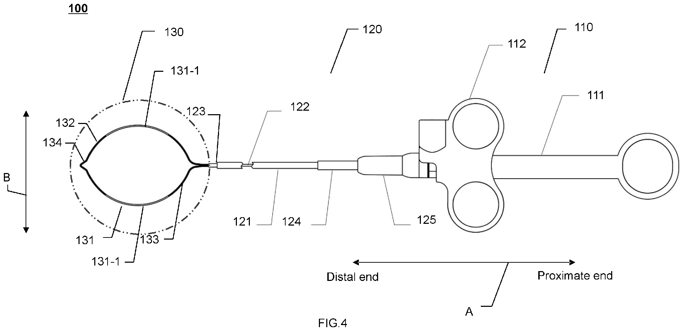

The initial stage to the third stage is a process in which the snare gradually tightens. The axial dimension is a maximum dimension of the snare portion along an axial direction A of a snare device 100 shown in FIG. 2 and FIG. 4. The radial dimension is a maximum dimension of the snare portion along a radial direction B of the snare device 100 shown in FIG. 2 and FIG. 4. Merely by way of example, when the snare portion is an elliptical snare as shown in FIG. 2, the axial dimension of the snare portion is a dimension corresponding to a major axis along the axial direction A, and the radial dimension is a dimension corresponding to a minor axis along the radial direction B.

According to the above table, the diameter-length ratio of the snare portion continuously decreases during a process of entering the sheath tube, causing the snaring part to gradually become slender. As a result, the lesion may not be completely enveloped by the slender snare. According to some embodiments of the present disclosure, the snare portion of the snare device is set to include a plurality of portions with different stiffnesses, that is, a first rigid portion and a second rigid portion. The first rigid portion is connected to the second rigid portion, at least a part of the structure of the second rigid portion is located at a distal end relative to the first rigid portion, and a stiffness of the first rigid portion is less than a stiffness of the second rigid portion. As the stiffnesses of the rigid portions are different, deformations of the rigid portions may be different during the operation of the snare device, thereby ensuring stable changes in a shape of the snaring part, avoiding the snare portion from gradually becoming slender, ensuring an integrity of the lesion enveloped by the snaring part, and improving efficiency and completeness of the cutting. At the same time, a phenomenon of easy slippage between the snare portion and the lesion may be avoided, making the contact between the snare portion and the lesion more fitting and stable.

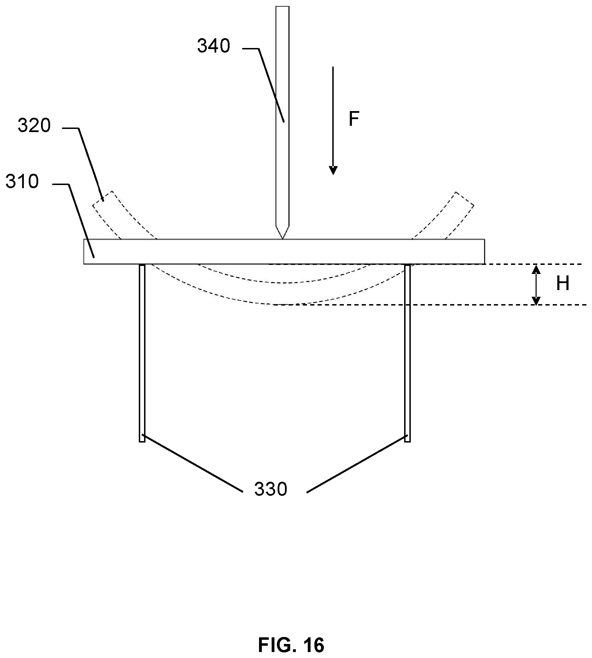

It is worth noting that the stiffness of each rigid portion described in the present disclosure is determined in a plurality of manners, for example, a tensile test manner, a compression test manner, a bending test manner, a torsion test manner, etc. As another example, the stiffness of each rigid portion is also determined by testing using the manner shown in FIG. 16. As shown in FIG. 16, a material 310 may be placed on a support member 330. A force application member 340 may apply a vertically downward force F toward a center position where the material 310 is placed. The material 310 deforms under an action of the force F and a deformed material 320 is obtained. A deformation magnitude between the material 310 and the deformed material 320 along a direction of the force F is a displacement H. A stiffness K of the material 310 may be calculated by the formula $K=F/H$. In addition, the hardness described in the present disclosure may also be determined by a plurality of manners, for example, a Rockwell hardness test manner, a Vickers hardness test manner, a Brinell hardness test manner, etc.

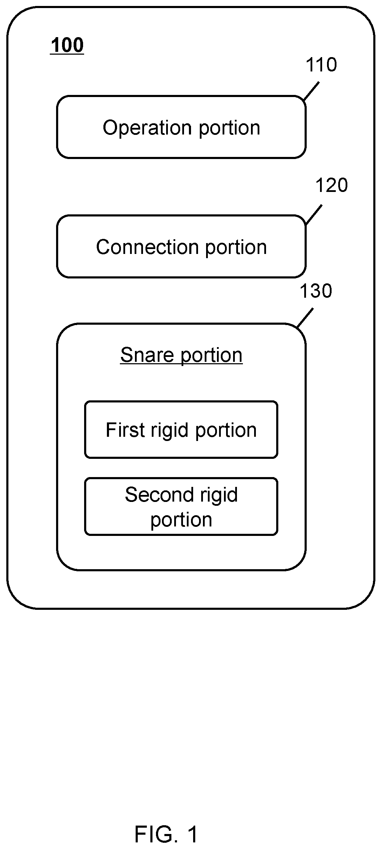

FIG. 1 is a module diagram illustrating an exemplary snare device according to some embodiments of the present disclosure.

The snare device 100 may be used to remove a lesion (e.g., a polyp). As shown in FIG. 1, the snare device 100 may include an operation portion 110, a connection portion 120, and a snare portion 130.

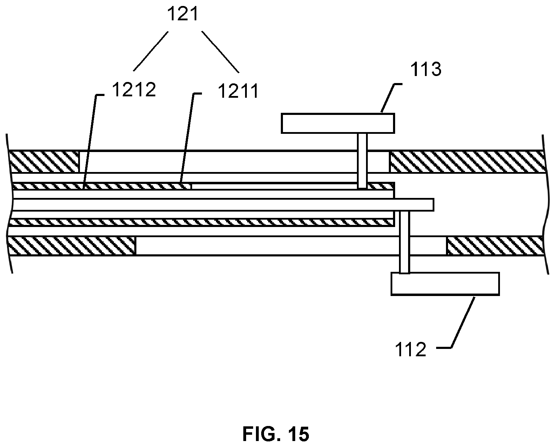

The operation portion 110 may be used for an operator (e.g., a doctor or a nurse using the snare device 100) to control the snare device 100. The operation portion 110 may be connected to the snare portion 130 and drive a part or all of the snare portion 130 to extend out of or enter the connection portion 120. The operation portion 110 may include a handle 111 and an adjustment member. The handle 111 may be configured for an operator to hold. The adjustment member may be configured to control the snare device 100. The adjustment member may include a first adjustment member. The first adjustment member may drive a part or all of the snare portion 130 to enter or extend out of the connection portion 120. For example, the first adjustment member includes a first slide member 112 as shown in FIG. 2. The first slide member 112 slides relative to the handle 111. When using the snare device 100, an operator may insert a finger into a limit hole of the first slide member 112 and apply a force along the axial direction A of the snare device 100 to the first slide member 112, so that the first slide member 112 slides relative to the handle 111 along the axial direction A. As another example, the first adjustment member further includes a second slide member 113 as shown in FIG. 15. The second slide member 113 also slides relative to the handle 111. The sliding of the second slide member 113 drives the sheath tube 121 to slide along the axial direction A of the snare device 100. More descriptions regarding the first slide member 112 and the second slide member 113 may be found in related descriptions later in the present disclosure.

The connection portion 120 may be configured to accommodate at least part of the snare portion 130. The connection portion 120 is further configured to connect the operation portion 110 and the snare portion 130. As shown in FIG. 2, the connection portion 120 may include a sheath tube 121 and a first traction member 122. The first traction member 122 is disposed in the sheath tube 121. The first traction member 122 connects the snare portion 130 and the operation portion 110. The operation portion 110 drives a part or all of the snare portion 130 to extend out of or enter the sheath tube 121 through the first traction member 122. Merely by way of example, a proximal end of the first traction member 122 is connected to the first slide member 112. A distal end of the first traction member 122 is connected to the snare portion 130. The sliding of the first slide member 112 drives the first traction member 122 to move along the axial direction A, thereby driving a part or all of the snare portion 130 to extend out of or enter the sheath tube 121. More descriptions about the first traction member 122 may be found in related descriptions later in the present disclosure.

It is worth noting that, as shown in FIG. 2, in the present disclosure, the distal end is an end farther from the operation portion 110 of the snare device 100, and the proximal end is an end closer to the operation portion 110 of the snare device 100.

In some embodiments, the connection portion 120 further includes other structures. As shown in FIG. 2, the connection portion 120 further includes a connection tube 123. A proximal end of the connection tube 123 is connected to the distal end of the first traction member 122. A distal end of the connection tube 123 is connected to the snare portion 130. The connection tube 123 may be disposed in the sheath tube 121 or outside the sheath tube 121. The connection portion 120 may further include a support tube 124. The support tube 124 may be disposed outside the sheath tube 121. It may be understood that when the sheath tube 121 is made of a flexible material, the support tube 124 may support the sheath tube 121 to ensure a normal use of the snare device 100. The connection portion 120 may further include a connection member 125. The sheath tube 121 may be connected to the operation portion 110 through the connection member 125.

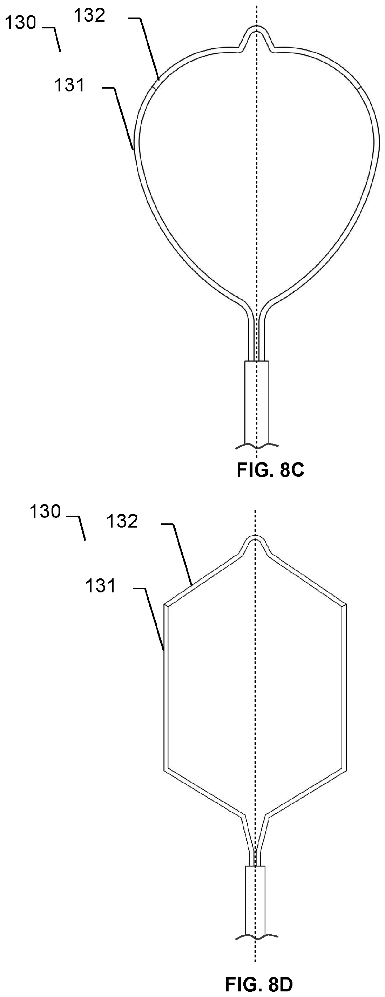

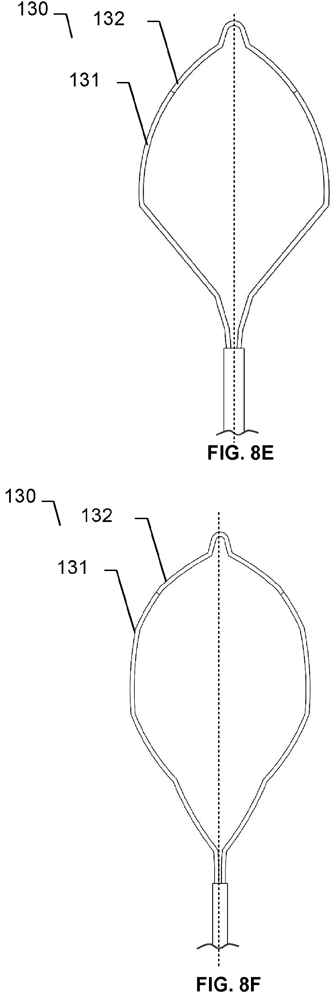

The snare portion 130 may be configured to resect a specified part of a patient. The snare portion 130 may be a ring-shaped structure. For example, a shape of the snare portion 130 is a circular ring-shaped structure, an elliptical ring-shaped structure, a heart-shaped ring-shaped structure, a shield-shaped ring-shaped structure, a hexagonal ring-shaped structure, or other polygonal ring-shaped structures, etc. The snare portion 130 may include one or more straight segment structures. For example, a hexagonal snare portion 130 shown in FIG. 8D includes a plurality of straight segment structures. The snare portion 130 may further include one or more arc-shaped structures. For example, a heart-shaped snare portion 130 shown in FIG. 8C includes a plurality of arc-shaped structures. As another example, a shield-shaped snare portion 130 shown in FIG. 8E includes a plurality of arc-shaped structures and a plurality of straight segment structures. More descriptions regarding the arc-shaped structure may be found in the following description of the present disclosure.

In some embodiments, the snare portion 130 includes a first rigid portion and a second rigid portion. The first rigid portion is connected to the second rigid portion. At least part of the second rigid portion is located at the distal end relative to the first rigid portion. A stiffness of the first rigid portion is less than a stiffness of the second rigid portion. In some embodiments, the first rigid portion includes a first connection segment 131. The second rigid portion includes a second connection segment 132. In some embodiments, the second rigid portion further includes a third connection segment 133. More description about the first connection segment 131, the second connection segment 132, and the third connection segment 133 may be found in the following description of the present disclosure.

The snare portion 130 may be formed from a variety of biocompatible materials. For example, a material of the snare portion 130 includes, but is not limited to, metal, polymer, alloy, etc. Further, the material of the snare portion 130 includes steel, tungsten, Nitinol, or titanium, etc. The material of the snare portion 130 may refer to a material of the first connection segment 131 and/or the second connection segment 132 in the embodiment of FIG. 2, or may refer to a material of the first connection segment 131, the second connection segment 132, and/or the third connection segment 133 in the embodiment of FIG. 4.

According to some embodiments of the present disclosure, a stiffness of a distal end of the snare portion 130 is increased by setting the snare portion 130 to include the first rigid portion and the second rigid portion with different stiffnesses, thereby avoiding the snare portion 130 from becoming slender during operation and ensuring fitting and capturing of the lesion.

In some embodiments, the first rigid portion and/or the second rigid portion may be configured with a marker, so that the operator can understand a stiffness difference of the snare portion more intuitively and make an adjustment as needed. For example, the first rigid portion and the second rigid portion are configured to have different colors and/or reflectivities.

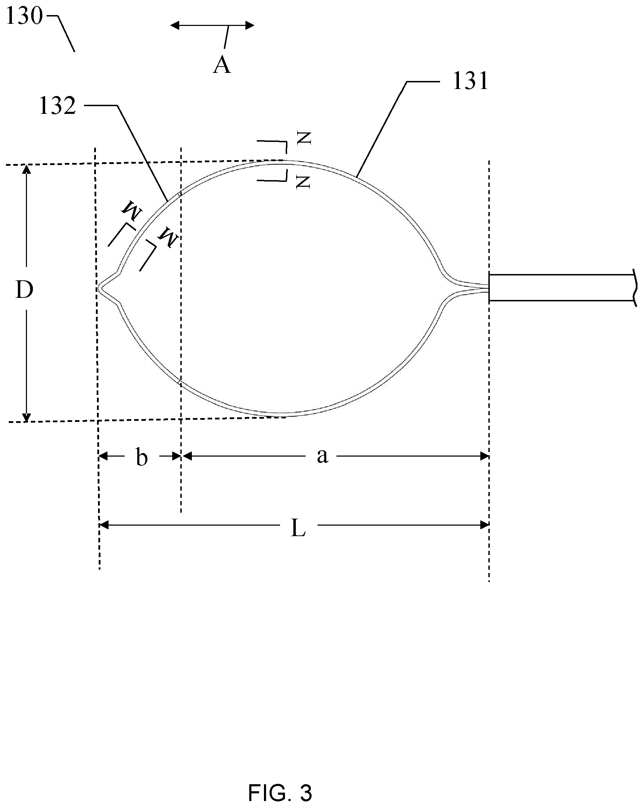

In some embodiments, when the second rigid portion only includes the second connection segment 132, and the snare portion 130 does not enter the connection portion 120, a ratio range of a dimension of the second connection segment 132 along the axial direction of the snare device 100 to a dimension of the snare portion 130 along the axial direction of the snare device 100 is 0.1-0.35, preferably 0.125-0.25. It is worth noting that, as shown in FIG. 3, the dimension of the second connection segment 132 along the axial direction A of the snare device 100 is b, and the dimension of the snare device 100 along the axial direction A is L. On one hand, when a ratio of b to L is less than 0.1, even if the second connection segment 132 with a greater stiffness is provided, since the dimension of the second connection segment 132 along the axial direction A of the snare device 100 is too small, the second connection segment 132 has little effect on the deformation of the entire snare portion 130 during the operation of the snare device 100, and the snare portion 130 may still become slender. On the other hand, when the ratio of b to L is greater than 0.35, since the dimension of the second connection segment 132 with the greater stiffness along the axial direction A of the snare device 100 is too great, the snare portion 130 may not fit and fix well with the lesion. After the first connection segment 131 enters the sheath tube 121, since the snaring part only includes the second connection segment 132, the snare portion 130 may still become slender during subsequent use. Preferably, when the ratio of b to L is one sixth, the snare portion 130 changes most stably and fits well with the lesion.

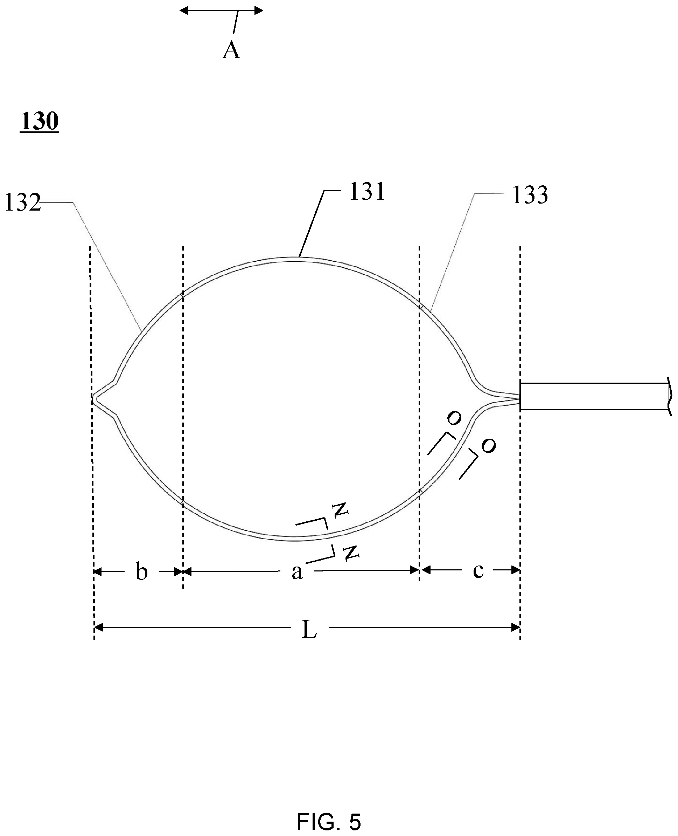

In some embodiments, when the second rigid portion further includes the third connection segment 133, and the snare portion 130 does not enter the connection portion 120, a ratio range of a dimension of the third connection segment 133 along the axial direction A of the snare device 100 to the dimension of the snare portion 130 along the axial direction A of the snare device 100 is 1/10-2/5, preferably 1/5-1/3. As shown in FIG. 5, the dimension of the third connection segment 133 along the axial direction A of the snare device 100 is c, and the dimension of the snare device 100 along the axial direction A is L. On one hand, when a ratio of c to L is less than 1/10, the snare has an insufficient stiffness and a poor wall adherence. On the other hand, when a ratio of c to L is greater than 2/5, a state with a small diameter-length ratio may occur during a contraction process, and the snare portion 130 may become slender.

Preferably, when a ratio of b, a, and c is 1:2:1, the snare portion 130 changes most stably and fits well with the lesion.

By limiting the dimensions of the first rigid portion and the second rigid portion along the axial direction A of the snare device 100, a stability of changes during use of the snare device 100 may be maintained, the snare portion 130 may be prevented from becoming slender during operation, and the fitting and capturing of the lesion may be ensured.

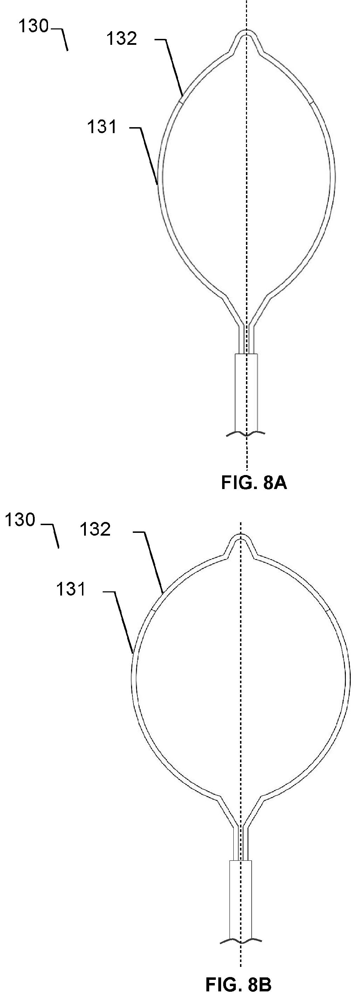

In some embodiments, the snare portion 130 is a symmetrical structure with an axis where the axial direction A of the snare device 100 is located as a symmetry axis. For example, the snare portion 130 is a symmetrical structure, such as an oval snare portion 130 shown in FIG. 8A, a circular snare portion 130 shown in FIG. 8B, a heart-shaped snare portion 130 shown in FIG. 8C, a hexagonal snare portion 130 shown in FIG. 8D, a shield-shaped snare portion 130 shown in FIG. 8E, a polygonal snare portion 130 shown in FIG. 8F, etc. The snare portions 130 of different shapes may be applicable to different application scenarios. For example, the oval snare portion 130 shown in FIG. 8A is used to snare common lesions (e.g., typical polyps). The circular snare portion 130 shown in FIG. 8B has a relatively large dimension along a radial direction (e.g., the radial direction B shown in FIG. 2), which facilitates approaching the lesion and is used to capture a great flat lesion. As another example, the hexagonal snare portion 130 shown in FIG. 8D is used to snare a flat lesion. By configuring the snare portion 130 as the symmetrical structure, the deformation of the snare portion 130 during use is stable, and the lesion may be more completely enveloped. In some embodiments, the second rigid portion is a symmetrical structure with an axis along an axial direction of the snare device 100 as a symmetry axis.

In some embodiments, the snare portion 130 also has other shapes. For example, the snare portion 130 is crescent-shaped. The crescent-shaped snare portion 130 facilitates an adjustment of an opening amplitude of the crescent-shaped snare portion 130 along the radial direction (e.g., the radial direction B shown in FIG. 2). In addition, the snare portion 130 is used with a transparent cap.

In some embodiments, the snare portion 130 further includes other structures. For example, a guide structure 134 is disposed on the snare portion 130. The guide structure 134 may be used to guide and position the lesion and may be V-shaped. The guide structure 134 may be disposed at a foremost end of the snare device 100 along the axial direction A. As shown in FIG. 1, the guide structure 134 may be disposed at the distal end of the second connection segment 132.

In some embodiments, when the snare portion 130 does not enter or partially enters the connection portion 120, a ratio range of the diameter-length ratio of the snare portion 130 is 0.4-1.1. The diameter-length ratio refers to a ratio of a dimension of the snaring part of the snare portion 130 along a radial direction of the snare device 100 to a dimension of the snaring part along the axial direction A of the snare device 100. It may be understood that the snaring part refers to a part of the snare portion 130 located outside the connection portion 120 (e.g., the sheath tube 121 in the connection portion 120). When the snare portion 130 does not enter the connection portion 120, the snaring part may be the entire snare portion 130. As shown in FIG. 3, when the snare portion 130 does not enter the connection portion 120, the diameter-length ratio is D/L, where D denotes a dimension of the snaring part along the radial direction of the snare device 100, and L denotes a dimension of the snaring part along the axial direction A of the snare device 100. Preferably, when the snare portion 130 does not enter or partially enters the connection portion 120, the ratio range of the diameter-length ratio of the snare portion 130 is 0.5-1. If the diameter-length ratio of the snare portion 130 is too great or too small, the snare portion 130 may become slender in the radial direction or the axial direction. For example, when the diameter-length ratio of the snare portion 130 is 0.2, it indicates that L of the snare portion 130 is excessively greater than D, and the snare portion 130 becomes slender in the axial direction.

The ratio range of the diameter-length ratio may be different for the snare portions 130 of different shapes. For example, when the shape of the snare portion 130 is oval, the diameter-length ratio of the snare portion 130 may be 0.5. As another example, when the shape of the snare portion 130 is circular, the diameter-length ratio of the snare portion 130 is 1. By setting different ratio ranges of the diameter-length ratio for snare portions 130 of different shapes, the stability of changes of the snare portion 130 in the axial direction and the radial direction during operation may be ensured, and the snare portion 130 may be prevented from becoming excessively slender, which affects use.

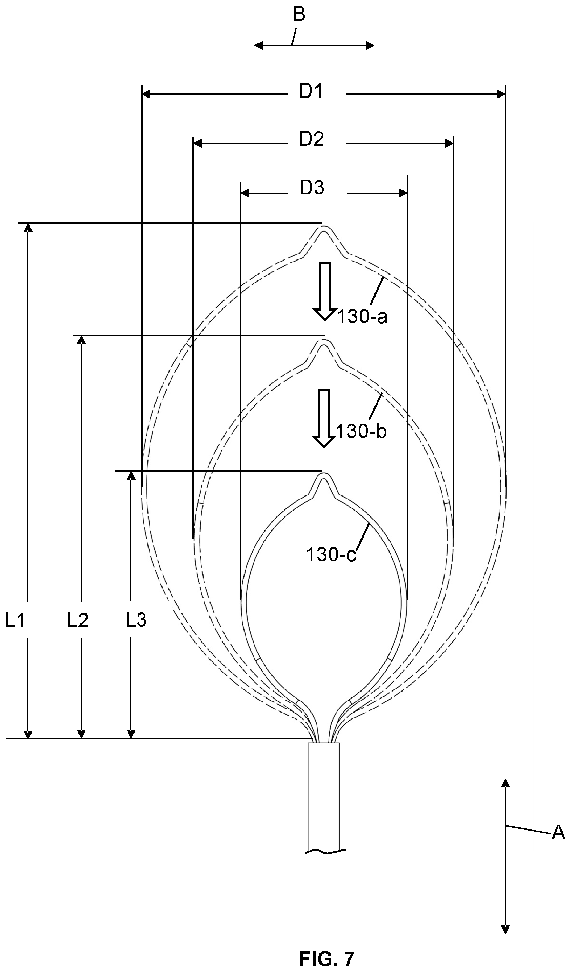

As shown in FIG. 7, during a tightening process of the snare portion 130, the snaring part located outside the connection portion 120 may change from a snaring part 130-a to a snaring part 130-b and then to a snaring part 130-c, where L1, L2, and L3 denote dimensions of the snaring part 130-a, the snaring part 130-b, and the snaring part 130-c along the axial direction A, respectively, and D1, D2, and D3 denote dimensions of the snaring part 130-a, the snaring part 130-b, and the snaring part 130-c along the radial direction B, respectively. During the tightening process of the snare portion 130, D1/L1, D2/L2, and D3/L3 are always not less than 0.5 and not greater than 1. Preferably, the ratio may be 0.7. Through the foregoing configuration in some embodiments of the present disclosure, the snare portion 130 is prevented from gradually becoming slender due to an excessively great dimension along the axial direction A or gradually becoming flat due to an excessively large dimension along the radial direction B during operation, thereby ensuring the stability of changes of the snare portion 130.

In some embodiments, when the snare portion 130 partially enters the connection portion 120, a first change rate of the snaring part of the snare portion 130 is not greater than a second change rate. The first change rate refers to a ratio of a change of a dimension of the snaring part along the radial direction B of the snare device 100 to an initial radial dimension. The second change rate refers to a ratio of an amount of change of a dimension of the snaring part along the axial direction A of the snare device 100 to an initial axial dimension. The initial radial dimension and the initial axial dimension are initial values of dimensions of the snare portion 130 along the radial direction B and the axial direction A, respectively, when the snare portion 130 does not enter the connection portion 120. In some embodiments, for any moment during operation of the snare device 100, the following formula (1) is satisfied:

$$\frac{(D-D_i)}{D} \leq \frac{(L-L_i)}{L} \qquad (1)$$

where $D$ denotes the initial radial dimension of the snare portion 130, $L$ denotes the initial axial dimension of the snare portion 130, $D_i$ denotes the dimension of the snaring part along the radial direction B of the snare device 100 at a current moment, and $L_i$ denotes the dimension of the snaring part along the axial direction A of the snare device at the current moment.

In some embodiments of the present disclosure, by limiting a magnitude relationship between the first change rate and the second change rate, the radial change of the snare portion 130 may be set smaller than the axial change, so as to prevent the snare portion 130 from gradually becoming slender, which is conducive to snaring and fixing the lesion.

In some embodiments, the first rigid portion includes a first connection segment 131, and the second rigid portion includes a second connection segment 132. FIG. 2 is a schematic diagram illustrating a snare device according to some embodiments of the present disclosure.

As shown in FIG. 2 and FIG. 3, the snare portion 130 may include the first connection segment 131 and the second connection segment 132, and the second connection segment 132 is located at a distal end relative to the first connection segment 131. The second connection segment 132 and the first connection segment 131 may be symmetrical structures with an axis along an axial direction of the snare device 100 as a symmetry axis, or may be asymmetrical structures. For example, the second connection segment 132 and the first connection segment 131 shown in FIG. 2 are both symmetrical structures with the axis along the axial direction of the snare device 100 as the symmetry axis. In this case, the entire second connection segment 132 is located at the distal end relative to the entire first connection segment 131. As another example, the second connection segment 132 and/or the first connection segment 131 may be the asymmetrical structures with the axis along the axial direction of the snare device 100 as the symmetry axis. For example, the second connection segment 132 on two sides of the axis of the snare device 100 have different lengths in the axial direction A, and/or the first connection segment 131 on two sides of the axis of the snare device 100 have different lengths in the axial direction A. In this case, a foremost distal end of the second connection segment 132 is located at a distal end (i.e., a distance to the operation portion 110 is greater) relative to a foremost distal end of the first connection segment 131.

In some embodiments, two ports at a distal end of the first connection segment 131 are connected to two ports at a proximal end of the second connection segment 132, respectively, thereby forming the snare portion 130. Two ports at a proximal end of the first connection segment 131 are connected to the operation portion 110. The first connection segment 131 and the second connection segment 132 may be strip-shaped structures. In some embodiments, the first connection segment 131 is a single strip-shaped structure. Any position of the strip-shaped structure other than the ports may be connected to the first traction member 122, and a connection point of the connection may be at the proximal end of the first connection segment 131. The two ports of the strip-shaped structure may be at a distal end of the first connection segment 131 and connected to the two ports at the proximal end of the second connection segment 132. A proximal end of the first traction member 122 is connected to the operation portion 110 (e.g., the first slide member 112 in the operation portion 110). In some embodiments, the first connection segment 131 includes two strip-shaped structures. As shown in FIG. 2, the first connection segment 131 includes two sub-connection segments 131-1. Two ports at distal ends of the two sub-connection segments 131-1 are connected to the two ports at the proximal end of the second connection segment 132. Two ports at proximal ends of the two sub-connection segments 131-1 are directly connected to the first traction member 122 or connected to the first traction member 122 through the connection tube 123. The proximal end of the first traction member 122 may be connected to the operation portion 110.

In some embodiments, a stiffness of the first connection segment 131 is less than a stiffness of the second connection segment 132. Preferably, a ratio range of the stiffness of the first connection segment 131 to the stiffness of the second connection segment 132 is 0.2-0.9, and preferably 0.3-0.8. For example, the stiffness of the first connection segment 131 is 50-400 N/m, and the stiffness of the second connection segment 132 is 200-1000 N/m. Preferably, the stiffness of the first connection segment 131 is 110 N/m, and the stiffness of the second connection segment 132 is about 300 N/m. By further limiting the range of stiffness of the first connection segment 131 and the range of stiffness of the second connection segment 132, the snare portion 130 is prevented from becoming slender while ensuring a smooth operation of the snare device 100 by an operator. In some embodiments, the stiffness of the first connection segment 131 being less than the stiffness of the second connection segment 132 is achieved through various configurations. More details on how to make the stiffness of the first connection segment 131 less than the stiffness of the second connection segment 132 is found in the following description of the present disclosure.

In some embodiments of the present disclosure, setting the stiffness of the second connection segment 132 located at the distal end of the snare portion 130 to be greater than the stiffness of the first connection segment 131 located at the proximal end of the second connection segment 132 can make, when the snare portion 130 is retracted into the connection portion 120, the second connection segment 132 at the distal end of the snare portion 130 provide more tension along the radial direction B to counteract a force along the axial direction A caused by retracting the snare portion 130, thereby reducing a radial deformation of the snare portion 130 during retraction, preventing the snare portion 130 from gradually becoming slender, and making it easier to ensnare a lesion. Furthermore, when the snare portion 130 contacts the lesion, the stiffness of the second connection segment 132 also provides a pressing force towards the lesion, thereby improving fit and stability between the snare portion 130 and a tissue.

As shown in some embodiments of the present disclosure, during operation of the snare device 100 in which the snare portion 130 includes the first connection segment 131 and the second connection segment 132, the changes of dimension of the snaring part as the snare tightens are shown in the table below.

| Stage | Radial dimension (mm) | Axial dimension (mm) | Diameter-length ratio |
|---|---|---|---|
| Initial stage | 27 | 42 | 0.64 |
| First stage | 20 | 38 | 0.53 |
| Second stage | 15 | 30 | 0.50 |
| Third stage | 10 | 20 | 0.50 |

It can be seen from the table above that, according to some embodiments of the present disclosure, by configuring the snare of the snare device 100 with the first connection segment 131 and the second connection segment 132 with different stiffnesses, a stable morphological change of the snare during operation of the snare device 100 can be ensured, thereby preventing the snare portion 130 from gradually becoming slender, ensuring an integrity of a lesion envelopment by the snare portion 130, and improving efficiency and completeness of cutting.

In some embodiments, the second rigid portion further includes the third connection segment 133. FIG. 4 is a schematic diagram illustrating another snare device according to some embodiments of the present disclosure.

As shown in FIG. 4 and FIG. 5, the snare portion 130 may include the first connection segment 131, the second connection segment 132, and the third connection segment 133. The third connection segment 133 is located at a proximal end relative to the first connection segment 131. The third connection segment 133 may be a symmetrical structure with an axis along an axial direction of the snare device 100 as a symmetry axis, or may be an asymmetrical structure. For example, both the third connection segment 133 and the first connection segment 131 shown in FIG. 4 are symmetrical structures with the axis along the axial direction of the snare device 100 as the symmetry axis, in this situation, the entire third connection segment 133 is located at a proximal end relative to the entire first connection segment 131. As another example, the third connection segment 133 and/or the first connection segment 131 are asymmetrical structures with the axis along the axial direction of the snare device 100 as the symmetry axis. For instance, portions of the third connection segment 133 on both sides of the axis of the snare device 100 have different lengths along the axial direction A, and/or portions of the first connection segment 131 on both sides of the axis of the snare device 100 have different lengths along the axial direction A. In this case, the most proximal end of the third connection segment 133 is located at a proximal end relative to the most proximal end of the first connection segment 131 (i.e., at a smaller distance from the operation portion 110).

In some embodiments, as shown in FIG. 4 and FIG. 5, two ports at a proximal end of the first connection segment 131 are connected to two ports at a distal end of the third connection segment 133, respectively, and two ports at the distal end of the first connection segment 131 are connected to two ports at a proximal end of the second connection segment 132, respectively. Two ports at a proximal end of the third connection segment 133 may be connected to the operation portion 110 via the first traction member 122. A port at a proximal end and a port at a distal end of one sub-connection segment 131-1 of the first connection segment 131 are connected to one port at the distal end of the third connection segment 133 and one port at the proximal end of the second connection segment 132, respectively, and a port at a proximal end and a port at a distal end of the other sub-connection segment 131-1 are connected to the other port at the distal end of the third connection segment 133 and the other port at the proximal end of the second connection segment 132, respectively.

In some embodiments, a stiffness of the first connection segment 131 is less than a stiffness of the second connection segment 132, and the stiffness of the first connection segment 131 is less than a stiffness of the third connection segment 133. For example, the stiffness of the first connection segment 131 is about 50-400 N/m, and the stiffness of the second connection segment 132 and the stiffness of the third connection segment 133 are about 200-1000 N/m. Preferably, the stiffness of the first connection segment 131 is 110 N/m, and both the stiffness of the second connection segment 132 and the stiffness of the third connection segment 133 are 300 N/m. In some embodiments, the stiffness of the first connection segment 131 is set less than the stiffness of the second connection segment 132 and the stiffness of the third connection segment 133 through various configurations. More details on how to configure the stiffness of the first connection segment 131 to be less than the stiffness of the second connection segment 132 and less than the stiffness of the third connection segment 133 may be found in the following description of the present disclosure.

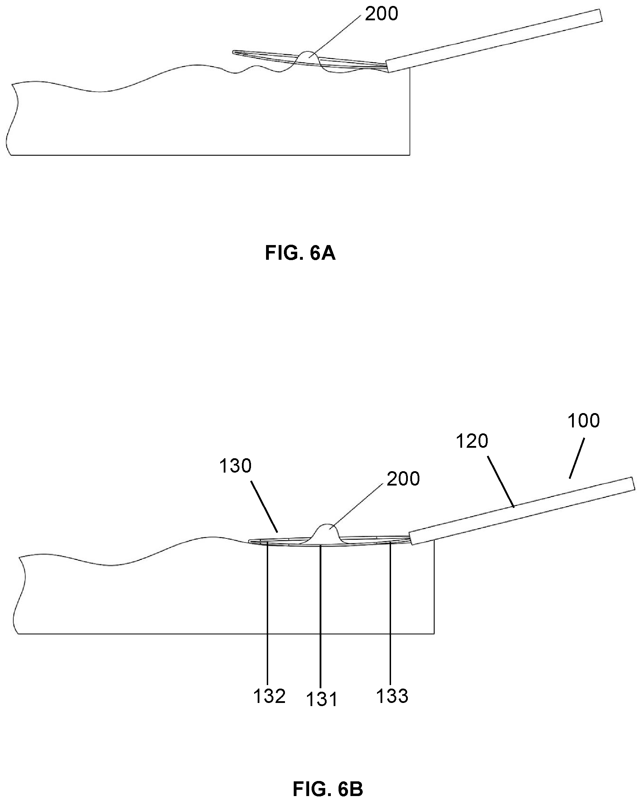

As shown in FIG. 6A, when a stiffness of a part of the snaring part of the snare device connected to the connection portion is relatively small, it is difficult for the snaring part to apply force to the lesion 200, and it is difficult to ensure a good fit of the snaring part of the snare device against the lesion 200. As shown in FIG. 6B, when the stiffness of the third connection segment 133 is relatively large, an operator may adjust a direction, an angle, etc., of the snare portion 130 when using the snare device 100, allowing the third connection segment 133 to apply force to the lesion 200, thereby causing a deformation of the lesion 200 and enabling the snare portion 130 of the snare device 100 to fit more closely against the lesion 200. According to some embodiments of the present disclosure, by providing the third connection segment 133 with a stiffness greater than the stiffness of the first connection segment 131 between the first connection segment 131 and the connection portion 120, a stable morphological change of the snare during the operation of the snare device 100 is ensured, thereby preventing the snare portion 130 from gradually becoming slender, and simultaneously ensuring that the snare portion 130 of the snare device 100 fits more closely against the lesion 200, improving the efficiency and completeness of cutting.

Furthermore, it may be understood that when the operator uses the snare device for snaring, if the stiffness of the part of the snare portion connected to the connection portion is small, and when the snare device is positioned in the air, a gravity influence of the snare portion may cause the snare portion to deform towards a gravity center of the snare portion, resulting in a shortened radial distance of the snare portion. However, in some embodiments of the present disclosure, by providing the third connection segment 133 with the stiffness greater than the stiffness of the first connection segment 131 between the first connection segment 131 and the connection portion 120, the deformation of the snare portion 130 in the air is reduced, thereby preventing the radial distance of the snare portion from shortening, and facilitating a snaring of the lesions by the snare device 100.

In some embodiments, the stiffness of the third connection segment 133 is the same as the stiffness of the second connection segment 132. By setting the stiffness of the third connection segment 133 to be the same as the stiffness of the second connection segment 132, the changes during use of the snare device 100 may be made uniform, thereby facilitating the control over the snaring progress by the operator. When the snare device 100 is used for snaring a polyp with a uniform overall hardness, the stiffness of the third connection segment 133 in the snare device 100 may be equal to the stiffness of the second connection segment 132, so that the snare portion 130 contacts and fits an overall tissue of the polyp. Preferably, when the stiffness of the third connection segment 133 is the same as the stiffness of the second connection segment 132, a ratio range of the stiffness of the first connection segment 131 to the stiffness of the second connection segment 132 is 0.2-0.9, and preferably is 0.3-0.8. Limiting the stiffness of the first connection segment 131 and the stiffness of the second connection segment 132 based on the ratio range makes the changes of the snare portion 130 along the axial direction A and the radial direction B more stable, thereby facilitating the control by the operator. For example, the stiffness of the first connection segment 131 is 310 N/m, and both the stiffness of the third connection segment 133 and the stiffness of the second connection segment 132 are 635 N/m.

In some embodiments, the stiffness of the third connection segment 133 is greater than the stiffness of the second connection segment 132. For example, the stiffness of the third connection segment 133 is 635 N/m, and the stiffness of the second connection segment 132 is 390 N/m. By setting the stiffness of the third connection segment 133 to be greater than the stiffness of the second connection segment 132, a shape of the snare device 100 can be maintained before a snaring operation is performed, deformation can be avoided, and snaring of the lesion can be facilitated. When the snare device 100 is used to snare a proximal polyp tissue that is relatively hard, the stiffness of the third connection segment 133 in the snare device 100 may be greater than the stiffness of the second connection segment 132, so that the snare portion 130 stably contacts a proximal end of the polyp. Preferably, when the stiffness of the third connection segment 133 is greater than the stiffness of the second connection segment 132, a ratio range of the stiffness of the second connection segment 132, the stiffness of the first connection segment 131, and the stiffness of the third connection segment 133 is (1.1-3.4):1:(1.4-3.6), and preferably (1.25-3.3):1:(1.5-3.5). Setting based on this ratio range may further improve a success rate of snaring by the snare device 100. It may be understood that the aforementioned ratio range needs to be set under the premise that the stiffness of the third connection segment 133 is greater than the stiffness of the second connection segment 132, that is, a value corresponding to the third connection segment 133 in the ratio range needs to be greater than a value corresponding to the second connection segment 132 in the ratio range.

In some embodiments, the stiffness of the third connection segment 133 is less than the stiffness of the second connection segment 132. For example, the stiffness of the third connection segment 133 is 390 N/m, and the stiffness of the second connection segment 132 is 635 N/m. By setting the stiffness of the third connection segment 133 to be less than the stiffness of the second connection segment 132, it is ensured that the operator can perform cutting with a relatively small force. When the snare device 100 is used to snare a distal polyp tissue that is relatively hard, the stiffness of the second connection segment 132 in the snare device 100 may be greater than the stiffness of the third connection segment 133, so that the snare portion 130 stably contacts a distal end of the polyp. Preferably, when the stiffness of the third connection segment 133 is less than the stiffness of the second connection segment 132, the ratio range of the stiffness of the second connection segment 132, the stiffness of the first connection segment 131, and the stiffness of the third connection segment 133 is (1.1-3.5):1:(1-3.2), and preferably (1.25-3.3):1:(1-3). Setting based on this ratio range may facilitate the cutting of the lesion by the operator, and reduce an operational difficulty. It may be understood that the aforementioned ratio range needs to be set under the premise that the stiffness of the third connection segment 133 is less than the stiffness of the second connection segment 132, that is, the value corresponding to the third connection segment 133 in the ratio range needs to be less than the value corresponding to the second connection segment 132 in the ratio range.

As shown in some embodiments of the present disclosure, during the operation of the snare device 100 in which the snare portion 130 includes the first connection segment 131, the second connection segment 132, and the third connection segment 133, as the snare tightens, dimensional changes of the snare portion are shown in the following table.

| Stage | Radial dimension (mm) | Axial dimension (mm) | Diameter-length ratio |
|---|---|---|---|
| Initial stage | 27 | 33.5 | 0.8 |
| First stage | 20 | 31 | 0.65 |
| Second stage | 15 | 21 | 0.71 |
| Third stage | 10 | 18 | 0.55 |

It can be seen from the above table that, in some embodiments of the present disclosure, by configuring the snare of the snare device 100 as the first connection segment 131, the second connection segment 132, and the third connection segment 133, and configuring the stiffness of the second connection segment 132 greater than the stiffness of the first connection segment 131, the snare morphology changes stably during the operation of the snare device 100, avoiding the snare portion 130 from gradually becoming slender, ensuring the completeness of the lesion envelopment by the snare portion 130, and improving the efficiency and completeness of cutting. By setting the stiffness of the third connection segment 133 located at the proximal end of the snare portion 130 to be greater than the stiffness of the first connection segment 131 located at the distal end of the third connection segment 133, when the snare portion 130 contacts and snares the lesion, the third connection segment 133 with a greater stiffness presses downward against a root of the lesion (the stiffness of the third connection segment 133 provides the pressing force toward the lesion), avoiding an easy slippage between the snare portion 130 and the lesion, thereby improving the fit, the stability, the success rate, and the accuracy of snaring. Of course, the stiffness of the third connection segment 133 may also reduce the radial deformation of the proximal end of the snare portion 130 during contraction, making it easier to snare tissue.

In some embodiments, the first rigid portion and the second rigid portion are an integrated structure or non-integrated structures. When the first rigid portion and the second rigid portion are non-integrated structures, the first rigid portion and the second rigid portion may be connected by various connection processes. The aforementioned connection processes may include, but not limited to, one or more of laser welding, brazing, plasma welding, argon arc welding, swaging, etc. Preferably, the first rigid portion and the second rigid portion may be connected by one or more of laser welding, brazing, or swaging, thereby increasing the stiffness at the connection between the first rigid portion and the second rigid portion, avoiding the snare portion 130 from becoming slender during operation, and ensuring a fitted snaring of the lesion. More descriptions regarding when the first rigid portion and the second rigid portion are an integrated structure, may be found in the relevant descriptions below of the present disclosure.

In some embodiments, at least a portion of one of the first rigid portion and the second rigid portion that is closer to the first traction member 122 is fixedly connected to the first traction member 122.

When the second rigid portion only includes the second connection segment 132, a structure of the one of the first rigid portion and the second rigid portion that is closer to the first traction member 122 is the first connection segment 131, and the first connection segment 131 may be fixedly connected to the first traction member 122. The proximal end of the first connection segment 131 may be directly or indirectly connected to the distal end of the first traction member 122. As shown in FIG. 2, the proximal end of the first connection segment 131 may be connected to the distal end of the connection tube 123, and the proximal end of the connection tube 123 may be connected to the distal end of the first traction member 122. Since the proximal end of the first traction member 122 is connected to the first slide member 112, sliding of the first slide member 112 may drive a part or all of the snare portion 130 to extend out of or enter the sheath tube 121, thereby causing the deformation of the snare portion 130.

When the second rigid portion further includes the third connection segment 133, the structure of the one of the first rigid portion and the second rigid portion that is closer to the first traction member 122 is the third connection segment 133, and the third connection segment 133 may be fixedly connected to the first traction member 122.

As shown in FIG. 4, the proximal end of the third connection segment 133 may be connected to the first traction member 122. In some optional embodiments, the proximal end of the third connection segment 133 forms an integrated structure with the first traction member 122. In this way, a production process of the third connection segment 133 and the first traction member 122 is simplified, the stability of the connection between the third connection segment 133 and the first traction member 122 is ensured, and a detachment of the third connection segment 133 is avoided. In other embodiments, the proximal end of the third connection segment 133 is fixedly connected to the first traction member 122. For example, the proximal end of the third connection segment 133 is fixedly connected by one or more of laser welding, brazing, or swaging with the first traction member 122. Since the proximal end of the third connection segment 133 may be connected to the first traction member 122, and the first traction member 122 may also be connected to the first slide member 112, the sliding of the first slide member 112 may drive a part or all of the snare portion 130 to extend out of or enter the sheath tube 121, thereby causing the deformation of the snare portion 130.

The following describes how to set the first rigid portion and the second rigid portion so that the stiffness of the first rigid portion is less than the stiffness of the second rigid portion. It may be understood that the snare device 100 adopts one or more of the following settings to ensure a stiffness difference between the first rigid portion and the second rigid portion. For example, a first feature dimension of the first connection segment 131 is the same as a second feature dimension of the second connection segment 132, but a hardness of the second connection segment 132 is greater than a hardness of the first connection segment 131, so that a stiffness of the second connection segment 132 is greater than a stiffness of the first connection segment 131. In addition, when the second rigid portion includes the second connection segment 132 and the third connection segment 133, a stiffness difference between the second connection segment 132 and the first connection segment 131 may be the same or different from a stiffness difference between the third connection segment 133 and the first connection segment 131.

In some embodiments, components of the first rigid portion and the second rigid portion may have a stiffness difference due to different hardnesses.

In some embodiments, a hardness of the second connection segment 132 is greater than a hardness of the first connection segment 131, to ensure that the stiffness of the first connection segment 131 is less than the stiffness of the second connection segment 132. For example, a difference between the hardness of the second connection segment 132 and the hardness of the first connection segment 131 is 5-10 HRB, the hardness of the second connection segment 132 is 80-95 HRB, and the hardness of the first connection segment 131 is 70-85 HRB. Preferably, the hardness of the second connection segment 132 may be 85-90 HRB, and the hardness of the first connection segment 131 may be 75-80 HRB, to reduce an operation difficulty for an operator while ensuring a uniform change of the snare portion 130.

The second connection segment 132 and the first connection segment 131 may be made of different materials, and during selection, the hardness of the first connection segment 131 is less than the hardness of the second connection segment 132. The second connection segment 132 and the first connection segment 131 may also be made of the same material. When the second connection segment 132 and the first connection segment 131 are made of the same material, the hardness of the second connection segment 132 may be increased through a hardening process (e.g., one or more of heat treatment, work hardening, alloying, fine grain strengthening, etc.).

Similarly, the hardness of the third connection segment 133 may also be greater than the hardness of the first connection segment 131, to ensure that the stiffness of the first connection segment 131 is less than the stiffness of the third connection segment 133.

In some embodiments, the components of the first rigid portion and the second rigid portion may have the stiffness difference due to different feature dimensions.

In some embodiments, the first feature dimension is less than the second feature dimension. The first feature dimension is a feature dimension of a cross-section of the first connection segment 131 perpendicular to an axial direction of the first connection segment 131, and the second feature dimension is a feature dimension of a cross-section of the second connection segment 132 perpendicular to an axial direction of the second connection segment 132. The aforementioned feature dimension may be a minimum dimension reflecting a shape characteristic of the cross-section. For example, when the cross-section is elliptical, dimensions reflecting characteristics of the cross-section may include a major axis and a minor axis, and the feature dimension is the minor axis. As another example, when the cross-section is rectangular, dimensions reflecting characteristics of the cross-section may include a length and a width, and the feature dimension can be a smaller one of the length and the width. As another example, when the cross-section is circular, a dimension reflecting characteristic of the cross-section may include a diameter, and the feature dimension is the diameter. As another example, when the cross-section is triangular, dimensions reflecting characteristics of the cross-section may include a height and a base, and the feature dimension is a smaller one of the height and the base.

In some embodiments, the first feature dimension and the second feature dimension may be of the same type. As shown in FIG. 3, the first feature dimension of a cross-section N-N of the first connection segment 131 perpendicular to the axial direction of the first connection segment 131 is less than the second feature dimension of a cross-section M-M of the second connection segment 132 perpendicular to the axial direction of the second connection segment 132. For example, when the cross-section N-N corresponding to the first connection segment 131 and the cross-section M-M corresponding to the second connection segment 132 are both circular, the first feature dimension and the second feature dimension are diameters of the cross-section N-N and the cross-section M-M, respectively, and the diameter of the cross-section M-M should be greater than the diameter of the cross-section N-N. In some embodiments, a difference between the second feature dimension and the first feature dimension is 0.1-0.2 mm. For example, diameters of the cross-section N-N and the cross-section M-M are 0.30 mm and 0.40 mm, respectively. As another example, diameters of the cross-section N-N and the cross-section M-M are 0.25 mm and 0.35 mm, respectively. As another example, diameters of the cross-section N-N and the cross-section M-M are 0.20 mm and 0.40 mm, respectively. Preferably, a ratio range of the first feature dimension to the second feature dimension is 0.5-0.95, and preferably 0.6-0.9.

In some embodiments, the first feature dimension and the second feature dimension may be of different types. For example, if the cross-section corresponding to the first connection segment 131 is circular, the first feature dimension is the diameter of the aforementioned circular cross-section, and if the cross-section corresponding to the second connection segment 132 is elliptical, the second feature dimension is the minor axis of the aforementioned elliptical cross-section.

Similarly, the first feature dimension is less than a third feature dimension, the third feature dimension is a feature dimension of a cross-section of the third connection segment 133 perpendicular to an axial direction of the third connection segment 133. In some embodiments, the first feature dimension and the third feature dimension are of the same type. For example, when a cross-section N-N corresponding to the first connection segment 131 and a cross-section O-O corresponding to the third connection segment 133 are both circular, the first feature dimension and the third feature dimension are diameters of the cross-section N-N and the cross-section O-O, respectively, and a diameter of the cross-section O-O should be greater than a diameter of the cross-section N-N. In some embodiments, the difference between the feature dimensions corresponding to the first feature dimension and the third feature dimension is 0.1-0.2 mm. For example, the diameters of the cross-section N-N and the cross-section O-O are 0.30 mm and 0.40 mm, respectively. Preferably, a ratio range of the first feature dimension to the third feature dimension is 0.5-0.95, and preferably 0.6-0.9.

In some embodiments, components of the first rigid portion and the second rigid portion have the stiffness difference due to different counts of minimum units.

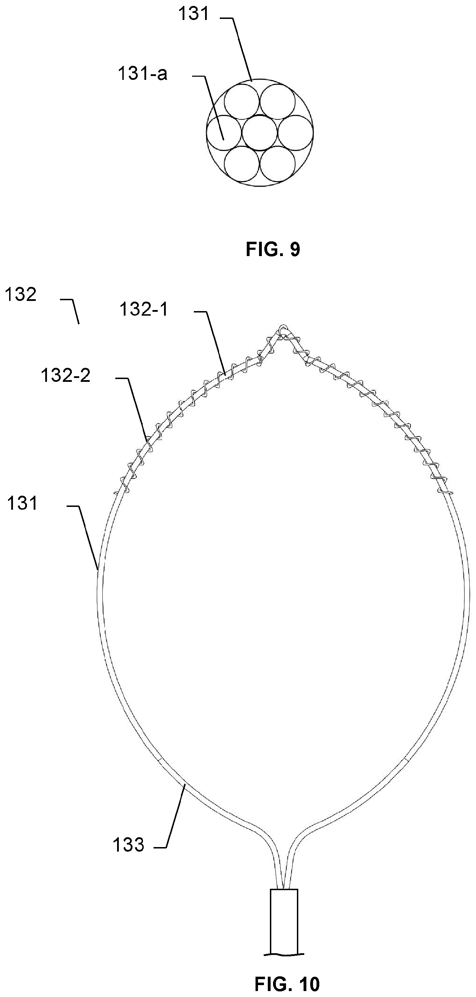

In some embodiments, when an absolute value of a difference among the feature dimensions is less than or equal to 0.1 mm (preferably 0.05 mm), each connection unit of a portion with a larger count of strands is thinner, and each connection unit of a portion with a smaller count of strands is thicker. As shown in FIG. 9, the first connection segment 131 includes at least two first connection units 131-a combined together, and the second connection segment 132 includes at least one second connection unit (not shown in the figure). The first connection unit 131-a and the second connection unit may be minimum units constituting the first connection segment 131 and the second connection segment 132, respectively. It may be understood that the first connection segment 131 and the second connection segment 132 are strip-shaped structures, and correspondingly, the first connection unit 131-a and the second connection unit are strip-shaped structures. Materials of the aforementioned first connection unit 131-a and the second connection unit may be one or more of nickel titanium, 304 stainless steel, or other feasible materials.

In some embodiments, a plurality of first connection units 131-a are combined through various combining processes to form the first connection segment 131. On any cross-section of the first connection segment 131 perpendicular to its axis, the plurality of first connection units are shown. As shown in FIG. 9, a certain first connection segment 131 includes 7 strands of first connection units 131-a. On any cross-section of the first connection segment 131 perpendicular to its axis, the 7 strands of first connection units 131-a are shown. For example, the plurality of first connection units 131-a are arranged and then rotated to cause deformation, thereby fixing the plurality of first connection units 131-a and generating the first connection segment 131. As another example, the plurality of first connection units 131-a are connected and fixed by welding, gluing, etc., to generate the first connection segment 131. Similarly, a plurality of second connection units are combined through the aforementioned various combining processes to form the corresponding second connection segment 132.

In some embodiments, a first count of strands is greater than a second count of strands, to ensure that the stiffness of the first connection segment 131 is less than the stiffness of the second connection segment 132. The first count of strands is a count of the first connection units 131-a included in the first connection segment 131, and the second count of strands is a count of the second connection units included in the second connection segment 132. For example, the second count of strands is 7 strands, and the first count of strands is 19 strands. As another example, the second count of strands is 3 strands, and the first count of strands is 7 strands.

It may be understood that, for example, when the first count of strands is 2 strands and the second count of strands is 1 strand, if the feature dimensions of the first connection unit 131-a and the second connection unit are the same, even though the first count of strands is greater than the second count of strands, in this case, the stiffness of the first connection segment 131 should be greater than the stiffness of the second connection segment 132. Therefore, when stiffnesses of different rigid portions are distinguished based on the count of strands, the feature dimensions of the different rigid portions need to be limited. To ensure that a difference in the count of strands is reflected as a stiffness difference, an absolute value of a difference in the feature dimensions between the first connection segment 131 and the second connection segment 132 is less than or equal to a first preset difference threshold. More preferably, except for the count of strands of the connection units (i.e., the first connection unit 131-a and the second connection unit), the first connection segment 131 and the second connection segment 132 are the same or similar. For example, differences between the feature dimensions, shapes of the cross-sections, physical and chemical properties of the first connection segment 131 and the second connection segment 132, etc. are 0 or within 5%. In some embodiments, when the difference between the first feature dimension and the second feature dimension is less than a first preset difference threshold, the first count of strands is greater than the second count of strands, so as to ensure that the stiffness of the first connection segment 131 is less than the stiffness of the second connection segment 132. The first preset difference threshold refers to a difference range between the first feature dimension and the second feature dimension when evaluating the first connection segment 131 and the second connection segment 132 based on the count of strands. In some embodiments, the first preset difference threshold is 0-0.1 mm, preferably any value in 0-0.05 mm. For example, the first preset difference threshold is 0. As another example, the first preset difference threshold is 0.02 mm or 0.05 mm.

In some embodiments, the third connection segment 133 includes at least one third connection unit, the first count of strands is greater than a third count of strands. The third count of strands is a count of strands of the third connection units included in the third connection segment 133. A material of the third connection unit may also be one or more of nickel titanium, 304 stainless steel, or other feasible materials. Similarly, to ensure that the difference in the count of strands is reflected as the difference in stiffness, the difference between the feature dimension of the first connection segment 131 and the feature dimension of the third connection segment 133 is less than or equal to a second preset difference threshold. More preferably, except for the count of strands of the connection units (i.e., the first connection unit 131-a and the third connection unit), the first connection segment 131 and the third connection segment 133 are the same or similar. For example, differences between the feature dimensions, shapes of the cross-sections, physical and chemical properties of the first connection segment 131 and the third connection segment 133, etc., are 0 or within 5%. In some embodiments, when the difference between the first feature dimension and the third feature dimension is less than the second preset difference threshold, the first count of strands is greater than the third count of strands, so as to ensure that the stiffness of the first connection segment 131 is less than the stiffness of the third connection segment 133. The second preset difference threshold refers to a difference range between the first feature dimension and the third feature dimension when evaluating the first connection segment 131 and the third connection segment 133 based on the count of strands. In some embodiments, the second preset difference threshold is 0-0.1 mm, preferably any value in 0-0.05 mm.

In some embodiments, the components of the first rigid portion and the second rigid portion have stiffness differences due to different cross-sectional shapes.

In some embodiments, a second cross-section is set as one or more of a circle, a square, or a hexagon, and a first cross-section is set as one or more of a semicircle, a triangle, or a rectangle. The first cross-section is a cross-section of the first connection segment 131 perpendicular to an axial direction of the first connection segment 131, and the second cross-section is a cross-section of the second connection segment 132 perpendicular to an axial direction of the second connection segment 132. It should be noted that, when materials, cross-sectional areas, and physical and chemical properties are the same or similar (e.g., a difference range is within 5%), as cross-sectional shapes such as the circle, the square, or the hexagon have more uniform dimensional distributions in all directions compared with cross-sectional shapes such as the semicircle, the triangle, or the rectangle, a stiffness of a structure with a cross-section set as the circle, the square, or the hexagon may be greater than a stiffness of a structure with a cross-section set as the semicircle, the triangle, or the rectangle.

Similarly, a third cross-section may be set as one or more of the circle, the square, or the hexagon, and the first cross-section may be set as one or more of the semicircle, the triangle, or the rectangle. The third cross-section is a cross-section of the third connection segment 133 perpendicular to an axial direction of the third connection segment 133.

By setting the third connection segment 133, the first connection segment 131, and the second connection segment 132 with different cross-sectional shapes, the third connection segment 133, the first connection segment 131, and the second connection segment 132 may have stiffness differences.

In some embodiments, the components of the first rigid portion and the second rigid portion have the stiffness differences due to stiffness reinforcement member(s).

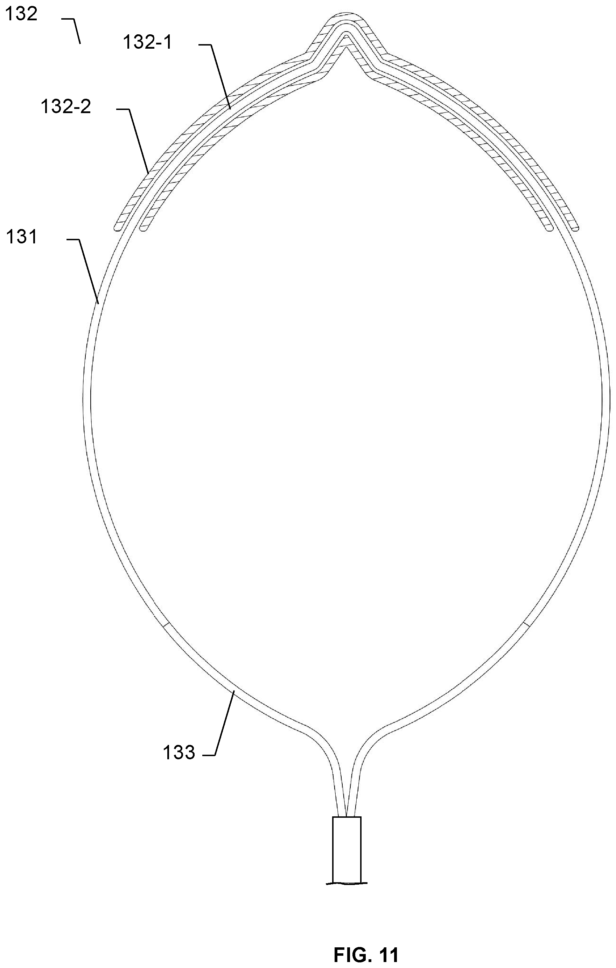

In some embodiments, the second connection segment 132 includes a base connection segment and a stiffness reinforcement member. The stiffness reinforcement member is fixed on the base connection segment. For example, the second connection segment 132 shown in FIG. 10 includes a base connection segment 132-1 and a stiffness reinforcement member 132-2. The stiffness reinforcement member 132-2 is helically wound around the base connection segment 132-1, thereby increasing the stiffness of the second connection segment 132. As another example, the second connection segment 132 shown in FIG. 11 includes the base connection segment 132-1 and the stiffness reinforcement member 132-2. The stiffness reinforcement member 132-2 is a tubular material sleeved outside the base connection segment 132-1. The tubular material may be a flexible material. A cross-section of the tubular material perpendicular to an axial direction thereof may have various shapes (e.g., a triangle, a rectangle, a square, etc.). For example, the stiffness reinforcement member further includes a medical coating disposed outside the base connection segment, and the medical coating increases the stiffness of the second connection segment 132.

In some embodiments, the stiffness of the stiffness reinforcement member is greater than the stiffness of the first connection segment 131. For example, the stiffness reinforcement member includes, but is not limited to, nickel titanium, titanium alloy, stainless steel, etc., with a stiffness greater than the stiffness of the first connection segment 131. According to some embodiments of the present disclosure, adjustments of a structure, a material, etc. of the stiffness reinforcement member can be facilitated by separately setting the base connection segment and the stiffness reinforcement member, thereby conveniently adjusting the stiffness of the second connection segment 132 as needed.

In some embodiments, the second connection segment 132 including the base connection segment and the stiffness reinforcement member is connected to the first connection segment 131 in various ways. For example, the base connection segment and the stiffness reinforcement member are combined, and two ports at the distal end of the first connection segment 131 are connected to two ports at a distal end of the combined base connection segment and stiffness reinforcement member in the second connection segment 132 through various connection processes (e.g., welding, adhesive bonding).

In some embodiments, the first connection segment 131 and the base connection segment are an integral structure. As shown in FIG. 10 and FIG. 11, the first connection segment 131 and the base connection segment 132-1 are an integral structure. The materials and the cross-sectional dimensions of the first connection segment 131 and the base connection segment may be the same, and the first connection segment 131 and the base connection segment are produced by integral molding to reduce production costs. In addition, a user of the snare device 100 may also flexibly select a position to set the stiffness reinforcement member according to actual needs (e.g., a shape of the lesion), so that the snare portion 130 fits the lesion more closely during use. According to some embodiments of the present disclosure, the first connection segment 131 and the base connection segment 132-1 are set as the integral structure, thereby enabling modification based on an existing snare device, setting the stiffness reinforcement member as needed, preventing the snare portion 130 from becoming slender during use, and also reducing the production cost of the modified snare device 100.

In some embodiments, similar to the second connection segment 132, the third connection segment 133 also includes a base connection segment and a stiffness reinforcement member. The stiffness reinforcement member is fixed on the base connection segment.

In some embodiments, the components of the first rigid portion and the second rigid portion have stiffness differences due to curvature(s) of arc-shaped structure(s).

As shown in FIG. 2, when both the first connection segment 131 and the second connection segment 132 include an arc-shaped structure, a curvature of the arc-shaped structure of the first connection segment 131 may be less than a curvature of the arc-shaped structure of the second connection segment 132, so as to increase the stiffness of the second connection segment 132, making the stiffness of the first connection segment 131 less than the stiffness of the second connection segment 132. Merely by way of example, a curvature radius of the first connection segment 131 is 15-55 mm, and a curvature radius of the second connection segment 132 is 6-17 mm. Preferably, the curvature radius of the first connection segment 131 is 20-50 mm, and the curvature radius of the second connection segment 132 is 8-15 mm. By further limiting the curvature radii of the two connection segments, it is ensured that the shape of the snare portion 130 facilitates capturing the lesion. Preferably, a ratio range of the curvature of the arc-shaped structure of the first connection segment 131 to the curvature of the arc-shaped structure of the second connection segment 132 may be 0.1-0.85, preferably 0.16-0.75.

In some embodiments of the present disclosure, the components of the first rigid portion and the second rigid portion have stiffness differences through one or more of the aforementioned settings. It should be noted that, when the stiffnesses of the second connection segment 132 and the third connection segment 133 are different, the stiffness difference is generated through similar settings.

In some embodiments, the snare portion 130 includes a base portion 1301 and an adjustment portion 1302. The adjustment portion 1302 is slidably connected to the base portion 1301.

The base portion 1301 may be configured to control an overall shape and size of the snare portion 130. Stiffness of parts in the base portion 1301 are the same.

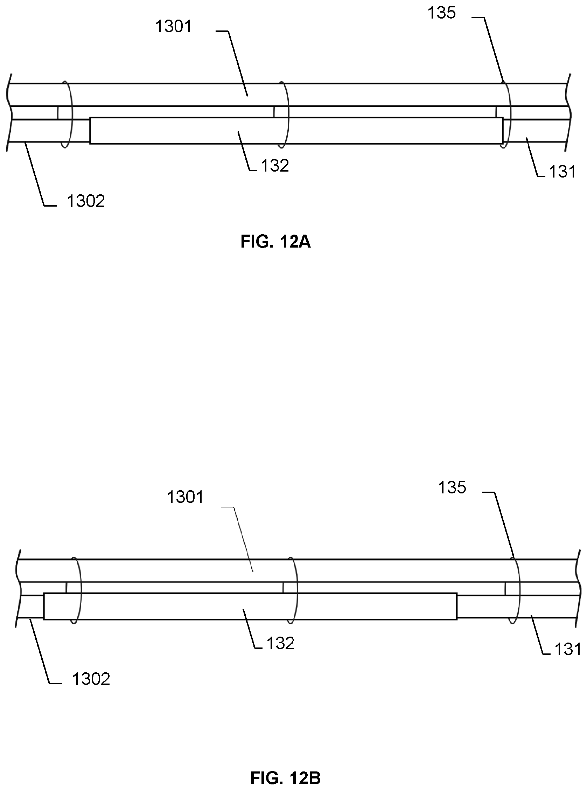

The adjustment portion 1302 is configured to adjust a stiffness of each position of the snare portion 130. The adjustment portion 1302 may include the first rigid portion and the second rigid portion. As shown in FIG. 12A, the adjustment portion 1302 may include the aforementioned first connection segment 131 and the second connection segment 132.

Sizes and shapes of the base portion 1301 and the adjustment portion 1302 are the same or similar, and they together constitute the snare portion 130. For example, the base portion 1301 and the adjustment portion 1302 differ only in that the base portion 1301 is provided with a guide structure 134. Two ports at a proximal end of the base portion 1301 are connected to a distal end of the first traction member 122. The operation portion 110 may control, via the first traction member 122, a part or all of the base portion 1301 to extend out of or enter the sheath tube 121. The materials of the base portion 1301 and the adjustment portion 1302 may be the same or different.

In some embodiments, the adjustment portion 1302 is slidable relative to the base portion 1301 to adjust relative positions of the first rigid portion and the second rigid portion in the snare portion 130, thereby achieving an adjustment of stiffness at various positions of the snare portion 130. FIG. 12A and FIG. 12B are schematic diagrams illustrating the same enlarged portion of the snare portion 130. An operator may adjust a position of the adjustment portion 1302 in the snare portion 130 shown in FIG. 12A. The adjustment portion 1302 may slide relative to the base portion 1301 and the snare portion 130 shown in FIG. 12B is obtained. As shown in FIG. 12B, compared with the positions of the first connection segment 131 and the second connection segment 132 of the adjustment portion 1302 in the snare portion 130 shown in FIG. 12A, the positions of the first connection segment 131 and the second connection segment 132 of the adjustment portion 1302 change.

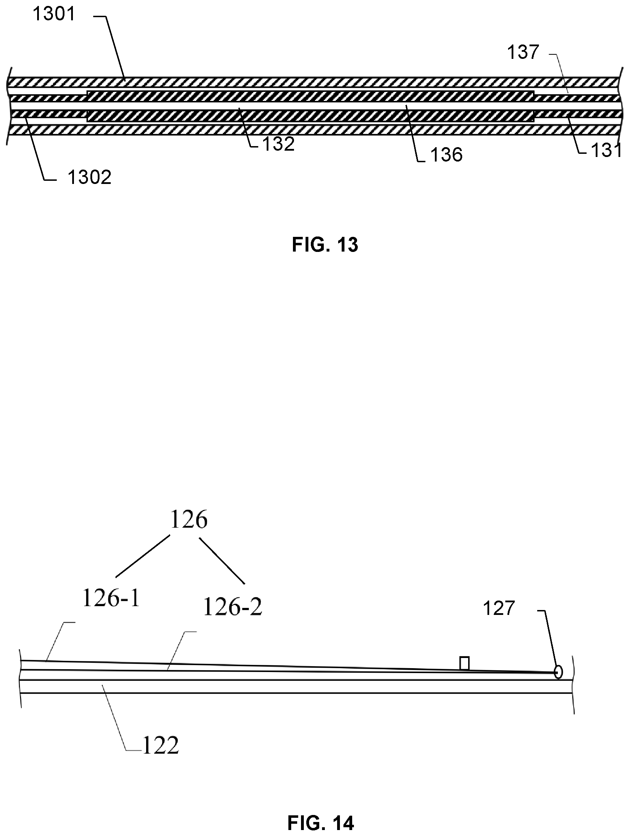

In some embodiments, the adjustment portion 1302 further includes a stiffness adjustment wire (not shown in the figures). As shown in FIG. 13, a first adjustment channel 136 is disposed in the adjustment portion 1302. The stiffness adjustment wire is detachably disposed in the first adjustment channel 136 to adjust the stiffness of the adjustment portion 1302.

In some embodiments, the adjustment portion 1302 and the base portion 1301 are arranged in various ways to enable the adjustment portion 1302 to slide relative to the base portion 1301.

In some embodiments, as shown in FIG. 12A, the base portion 1301 is provided with a plurality of connection rings 135. The adjustment portion 1302 passes through the connection rings 135 and is slidably connected to the base portion 1301. The base portion 1301 may be fixedly connected to the plurality of connection rings 135. The adjustment portion 1302 may slide relative to the base portion 1301 when driven by the operation portion 110. When the adjustment portion 1302 slides relative to the base portion 1301, positions of the base portion 1301 and the connection rings 135 do not change.

In some embodiments, as shown in FIG. 13, a second adjustment channel 137 is disposed in the base portion 1301. The adjustment portion 1302 is slidably disposed in the second adjustment channel 137. The operation portion 110 may drive the adjustment portion 1302 to slide within the second adjustment channel 137. When the adjustment portion 1302 slides within the second adjustment channel 137, the position of the base portion 1301 itself does not change.

In some embodiments, the operation portion 110 controls the adjustment portion 1302 to slide relative to the base portion 1301. The connection portion 120 may further include a second traction member 126. The second traction member 126 is disposed within the sheath tube 121. Both the second traction member 126 and the first traction member 122 are configured to be movable along the axial direction of the sheath tube 121. The second traction member 126 is slidable relative to the first traction member 122. The second traction member 126 may be connected to the adjustment portion 1302. For example, as shown in FIG. 14, the second traction member 126 includes a first pulling cable 126-1 and a second pulling cable 126-2. A port at a distal end of the first pulling cable 126-1 is connected to a port at a proximal end of the adjustment portion 1302. A port at a distal end of the second pulling cable 126-2 is connected to another port at the proximal end of the adjustment portion 1302. The operation portion 110 may control the first pulling cable 126-1 and the second pulling cable 126-2 to slide along the axial direction of the sheath tube 121, thereby driving the adjustment portion 1302 to slide relative to the base portion 1301.

In some embodiments, the operation portion 110 synchronously drives a part or all of the base portion 1301 and the adjustment portion 1302 to extend out of or enter the sheath tube 121. In some embodiments, a limiting structure is disposed on the first traction member 122. The limiting structure may limit a position of the second traction member 126 on the first traction member 122, enabling the operation portion 110 to synchronously drive a part or all of the base portion 1301 and the adjustment portion 1302 to extend out of or enter the sheath tube 121, while the second traction member 126 is slidable relative to the first traction member 122 within the limiting structure. For example, the limiting structure is a limit ring 127 as shown in FIG. 14. The limit ring 127 is fixedly connected to the first traction member 122. Ports at the proximal ends of the first pulling cable 126-1 and the second pulling cable 126-2 pass through a through hole in the limit ring 127 and are connected, and are able to slide within the through hole of the limit ring 127. Sliding of the first slide member 112 drives the first traction member 122 to move along the axial direction A, and simultaneously drives the limit ring 127 and the second traction member 126 disposed in the limit ring 127 to move along the axial direction A, thereby synchronously driving a part or all of the base portion 1301 and the adjustment portion 1302 to extend out of or enter the sheath tube 121. As another example, the limiting structure is a pulley. A rotation shaft of the pulley is fixedly disposed on the first traction member 122. The ports at the proximal ends of the first pulling cable 126-1 and the second pulling cable 126-2 are wound in a groove of the pulley. The sliding of the first slide member 112 drives the first traction member 122 to move along the axial direction A, simultaneously drives the pulley and the second traction member 126 disposed on the pulley to move along the axial direction A, thereby synchronously driving a part or all of the base portion 1301 and the adjustment portion 1302 to extend out of or enter the sheath tube 121.

In some embodiments, the adjustment member further includes a second adjustment member. The second adjustment member is used to drive the adjustment portion 1302 to slide relative to the base portion 1301. For example, when the limiting structure is the limit ring 127, the second adjustment member is a sliding block. A count of the sliding block can be one or two. The sliding block is fixedly connected to the first pulling cable 126-1 or the second pulling cable 126-2 and extends out of the handle 111. An operator slides the sliding block along the axial direction A of the snare device 100, drives the first pulling cable 126-1 and/or the second pulling cable 126-2 to slide along the axial direction A of the snare device 100, thereby driving the adjustment portion 1302 to slide relative to the base portion 1301. As the limit ring 127 is disposed on the first traction member 122, it ensures that the adjustment portion 1302 slides relative to the base portion 1301 without moving as a whole along the axial direction A. As another example, when the limiting structure is the pulley, the second adjustment member is a knob. The knob passes through the handle 111 and is connected to the pulley. The operator rotates the knob, drives the pulley to rotate, causing the first pulling cable 126-1 and the second pulling cable 126-2 to slide along the axial direction A of the snare device 100, thereby driving the adjustment portion 1302 to slide relative to the base portion 1301.

According to some embodiments of the present disclosure, the sliding of the adjustment portion 1302 relative to the base portion 1301 is achieved through one or more of the aforementioned arrangements. When an overall size of the snare portion 130 is determined, the operator may also use the adjustment portion 1302 to adjust the stiffnesses of different positions of the snare portion 130, thereby adjusting the shape of the snare portion 130 as needed to adapt to requirements of different scenarios.

In some embodiments, the first adjustment member controls the snare portion 130 to enter the connection portion 120 in various ways. The first adjustment member may be connected to the two ports at the proximal end of the snare portion 130 via the first traction member 122. The first adjustment member drives the two ports at the proximal end of the snare portion 130 to move synchronously or asynchronously.

In some embodiments, the first adjustment member includes the first slide member 112. The operator controls, via the first slide member 112, the first traction member 122 to move along the axial direction A, drives the snare portion 130 to move along the axial direction A, thereby causing a part or all of the snare portion 130 to extend out of or enter the sheath tube 121. For example, the first traction member 122 may include one strip-like structure (e.g., the first traction member 122 can be one pulling rope). Both ports at the proximal end of the snare portion 130 are connected to the distal end of the first traction member 122. When the operation portion 110 controls, via the first slide member 112, the first traction member 122 to move along axial direction A, the two ports at the proximal end of the snare portion 130 move synchronously, causing a part or all of the snare portion 130 to extend out of or enter the sheath tube 121. As another example, the first traction member 122 also includes two strip-shaped structures (e.g., the first traction member 122 is two pulling ropes), namely a first sub-traction member and a second sub-traction member. The first slide member 112 includes a first sub-slide member and a second sub-slide member. The first sub-slide member is connected to a proximal end of the first sub-traction member, and a distal end of the first sub-traction member is connected to one port at the proximal end of the snare portion 130. The second sub-slide member is connected to a proximal end of the second sub-traction member, and a distal end of

# EP 4 755 324 A1

the second sub-traction member is connected to the other port at the proximal end of the snare portion 130. The first sub-slide member and the second sub-slide member slide independently to respectively drive a corresponding port of the snare portion 130 to move along the axial direction A of the snare device 100, thereby achieving a asynchronous movement of the two ports at the proximal end of the snare portion 130, and causing a part or all of the snare portion 130 to extend out of or enter the sheath tube 121. For instance, one port at the proximal end of the snare portion 130 extends out of the sheath tube 121, while the other port enters the sheath tube 121. The aforementioned control of the two ports at the proximal end of the snare portion 130 via the first sub-slide member and the second sub-slide member allows a continuous adjustment of an expansion amplitude of the structures on both axial sides of the snare portion 130 when passing a head end of a large polyp, enabling a quick passage through tissue gaps. The operator may also adjust a snaring angle by controlling the two ports at the proximal end of the snare portion 130 according to the lesion, thereby improving a success rate of snaring.

According to some embodiments of the present disclosure, the two ports at the proximal end of the snare portion 130 are driven to move synchronously or asynchronously via the first adjustment member, allowing the operator to control the shape of the snare portion 130 more flexibly.

In some embodiments, the first adjustment member further includes the second slide member 113. The operator controls, via the second slide member 113, at least a part of the structure of the sheath tube 121 to move along the axial direction A, thereby driving a part or all of the snare portion 130 to extend out of or enter the sheath tube 121. As shown in FIG. 15, the sheath tube 121 may include an outer sheath tube 1211 and an inner sheath tube 1212. A position of the outer sheath tube 1211 in the snare device 100 remains unchanged. The inner sheath tube 1212 is movable relative to the outer sheath tube 1211 along the axial direction A of the snare device 100. A proximal end of the inner sheath tube 1212 may be fixedly connected to the second slide member 113. The second slide member 113 is slidable along the axial direction A of the snare device 100, and drives the inner sheath tube 1212 to move along the axial direction A, thereby driving a part or all of the snare portion 130 to extend out of or enter the sheath tube 121.

It may be understood that when snaring is performed based on the first slide member 112, a contraction of the snare portion 130 proceeds from the distal end towards the proximal end. A position of the sheath tube 121 remains unchanged, and the proximal end of the snare portion 130 continuously contracts into the sheath tube 121. However, an overall stiffness of the snare portion 130 is relatively small, and smaller lesions are prone to slipping off during snaring. When the snare device 100 is provided with the second slide member 113, the snare device 100 may perform snaring based on the second slide member 113. In this case, the position of the snare portion 130 remains unchanged, and the inner sheath tube 1212 moves along the axial direction A towards the distal end to adjust a size of the snare portion 130 for snaring the lesion. As a stiffness of the inner sheath tube 1212 is greater than the stiffness of the snare portion 130, the inner sheath tube 1212 may press against a root of the lesion during snaring, making even smaller lesions less likely to slip off, thereby improving the success rate of snaring. It may be understood that, as shown in FIG. 15, the snare device 100 simultaneously includes the first slide member 112 and the second slide member 113. The operator may select a corresponding component to operate as needed, thereby enhancing an operational flexibility of the snare device 100. For example, the operator performs snaring on the lesion via the second slide member 113, and then performs cutting on the lesion via the first slide member 112.

Having thus described the basic concepts, it is rather apparent to those skilled in the art that the foregoing detailed disclosure is intended to be presented by way of example only and is not limiting. Various alterations, improvements, and modifications may occur and are intended to those skilled in the art, though not expressly stated herein. These alterations, improvements, and modifications are intended to be suggested by the present disclosure, and are within the spirit and scope of the exemplary embodiments of the present disclosure.

Meanwhile, the present disclosure uses specific words to describe the embodiments thereof. For example, "one embodiment," "an embodiment," and/or "some embodiments" mean a certain feature, structure, or characteristic associated with at least one embodiment of the present disclosure. Therefore, it should be emphasized and noted that two or more references to "an embodiment," "one embodiment," or "an alternative embodiment" in various places in the present disclosure are not necessarily all referring to the same embodiment. In addition, certain features, structures, or characteristics in one or more embodiments of the present disclosure may be appropriately combined.

In addition, unless explicitly stated in the claims, the order of processing elements and sequences, the use of counts and letters, or the use of other names in the present disclosure are not intended to limit the sequence of processes and methods of the present disclosure. Although the foregoing disclosure discusses some embodiments of the invention currently considered useful through various examples, it should be understood that such details are for illustrative purposes only, and the appended claims are not limited to the disclosed embodiments. On the contrary, the claims are intended to cover all modifications and equivalent combinations that conform to the substance and scope of the embodiments of the present disclosure. For example, although the implementation of various components described above may be embodied in a hardware device, it may also be implemented as a software only solution, e.g., an installation on an existing server or mobile device.

Similarly, it should be noted that, in order to simplify the expression disclosed in the present disclosure and thereby facilitate the understanding of one or more embodiments of the present disclosure, various features are sometimes grouped into one embodiment, drawing, or description thereof in the foregoing description of the embodiments of the present disclosure. However, this manner of disclosure does not mean that the object of the present disclosure requires more features than those mentioned in the claims. Rather, claimed subject matter may lie in less than all features of a single foregoing disclosed embodiment.

In some embodiments, counts describing the quantity of components or properties are used. It should be understood that such counts used to describe the embodiments are modified by the modifiers "approximately," "about," or "substantially" in some examples. Unless otherwise stated, "approximately," "about," or "substantially" indicates that the stated count allows a variation of 20%. Accordingly, in some embodiments, the numerical parameters used in the present disclosure and claims are approximate values, which vary according to the desired characteristics of individual embodiments. In some embodiments, numerical parameters should consider the specified count of significant digits and adopt the method of general digit retention. Although the numerical ranges and parameters used to confirm the breadth of their scope in some embodiments of the present disclosure are approximate values, such numerical values are set as precisely as possible within the feasible range in specific embodiments.

Each patent, patent application, patent application publication, and other material, such as articles, books, specifications, publications, documents, etc., cited in the present disclosure is hereby incorporated by reference in its entirety into the present disclosure. This excludes application history documents that are inconsistent with or conflict with the content of the present disclosure, and also excludes documents that limit the broadest scope of the claims of the present disclosure (whether currently or subsequently appended to the present disclosure). It should be noted that if the description, definition, and/or use of terms in the ancillary materials of the present disclosure are inconsistent with or conflict with the content described in the present disclosure, the description, definition, and/or use of terms in the present disclosure shall prevail.

Finally, it should be understood that the embodiments described in the present disclosure are only used to illustrate the principles of the embodiments of the present disclosure. Other variations may also fall within the scope of the present disclosure. Therefore, by way of example and not limitation, alternative configurations of the embodiments of the present disclosure may be considered consistent with the teachings of the present disclosure. Accordingly, the embodiments of the present disclosure are not limited to the embodiments explicitly introduced and described in the present disclosure.

**Claims**

1. A snare device, comprising:

   a snare portion, including a first rigid portion and a second rigid portion, wherein the first rigid portion is connected to the second rigid portion, at least a portion of a structure of the second rigid portion is located at a distal end relative to the first rigid portion, and a stiffness of the first rigid portion is less than a stiffness of the second rigid portion;
   a connection portion, configured to accommodate at least a portion of the snare portion; and
   an operation portion, connected to the snare portion, the operation portion driving a portion or all of the snare portion to extend out of or enter the connection portion.

2. The snare device according to claim 1, wherein when the snare portion does not enter the connection portion or partially enters the connection portion, a range of a diameter-length ratio of the snare portion is 0.5-1, wherein the diameter-length ratio is a ratio of a dimension of a snaring part of the snare portion along a radial direction of the snare device to a dimension of the snaring part along an axial direction of the snare device.

3. The snare device according to claim 1, wherein during a process of the snare portion partially entering the connection portion, a first change rate of a snaring part of the snare portion is not greater than a second change rate of the snaring part, wherein

   the first change rate refers to a ratio of a change of a dimension of the snaring part along a radial direction of the snare device to an initial radial dimension; and
   the second change rate refers to a ratio of a change of a dimension of the snaring part along an axial direction of the snare device to an initial axial dimension.

4. The snare device according to claim 1, wherein

   the first rigid portion includes a first connection segment; and

the second rigid portion includes a second connection segment, wherein the second connection segment is located at a distal end relative to the first connection segment, and a stiffness of the first connection segment is less than a stiffness of the second connection segment.

5. The snare device according to claim 4, wherein

two ports at a distal end of the first connection segment are respectively connected to two ports at a proximal end of the second connection segment; and
two ports at a proximal end of the first connection segment are connected to the operation portion.

6. The snare device according to claim 4, wherein when the snare portion does not enter the connection portion, a ratio range of a dimension of the second connection segment along the axial direction of the snare device to a dimension of the snare portion along the axial direction of the snare device is 0.1-0.35.

7. The snare device according to claim 4, wherein a ratio range of the stiffness of the first connection segment to the stiffness of the second connection segment is 0.2-0.9.

8. The snare device according to claim 4, wherein both the first connection segment and the second connection segment include an arc-shaped structure, wherein a curvature of an arc-shaped structure of the first connection segment is less than a curvature of an arc-shaped structure of the second connection segment.

9. The snare device according to claim 8, wherein a ratio range of the curvature of the arc-shaped structure of the first connection segment to the curvature of the arc-shaped structure of the second connection segment is 0.1-0.85.

10. The snare device according to claim 4, wherein a hardness of the second connection segment is greater than a hardness of the first connection segment.

11. The snare device according to claim 4, wherein a first feature dimension is less than a second feature dimension, wherein

the first feature dimension is a feature dimension of a cross-section of the first connection segment perpendicular to an axial direction of the first connection segment; and
the second feature dimension is a feature dimension of a cross-section of the second connection segment perpendicular to an axial direction of the second connection segment.

12. The snare device according to claim 11, wherein a ratio range of the first feature dimension to the second feature dimension is 0.5-0.95.

13. The snare device according to claim 4, wherein

the first connection segment includes at least two first connection units, and the second connection segment includes at least one second connection unit; and
a first count of strands is greater than a second count of strands, wherein the first count of strands is a count of first connection units of the first connection segment, and the second count of strands is a count of a second connection unit of the second connection segment.

14. The snare device according to claim 13, wherein when an absolute value of a difference between a first feature dimension and a second feature dimension is less than or equal to a first preset threshold, the first count of strands is greater than the second count of strands, wherein

the first feature dimension is a feature dimension of a cross-section of the first connection segment perpendicular to an axial direction of the first connection segment; and
the second feature dimension is a feature dimension of a cross-section of the second connection segment perpendicular to an axial direction of the second connection segment.

15. The snare device according to claim 14, wherein the first preset threshold is any value between 0mm and 0.1mm.

16. The snare device according to claim 4, wherein a shape of a second cross-section is a circle, a square, a hexagon, or a combination thereof, and a shape of a first cross-section is a semicircle, a triangle, a rectangle, or a combination thereof, wherein

the first cross-section is a cross-section of the first connection segment perpendicular to an axial direction of the first connection segment; and

the second cross-section is a cross-section of the second connection segment perpendicular to an axial direction of the second connection segment.

17. The snare device according to claim 4, wherein the second connection segment includes a base connection segment and a stiffness reinforcement member, the stiffness reinforcement member being fixed on the base connection segment.

18. The snare device according to claim 17, wherein the first connection segment and the base connection segment are an integral structure.

19. The snare device according to any one of claims 4 to 18, wherein the second rigid portion further includes a third connection segment, wherein

the third connection segment is located at a proximal end relative to the first connection segment;

the stiffness of the first connection segment is less than the stiffness of the second connection segment; and

the stiffness of the first connection segment is less than a stiffness of the third connection segment.

20. The snare device according to claim 19, wherein

the two ports at the distal end of the first connection segment are respectively connected to the two ports at the proximal end of the second connection segment;

the two ports at the proximal end of the first connection segment are respectively connected to two ports at a distal end of the third connection segment; and

two ports at a proximal end of the third connection segment are connected to the operation portion.

21. The snare device according to claim 19, wherein when the snare portion does not enter the connection portion, a ratio range of a dimension of the third connection segment along the axial direction of the snare device to the dimension of the snare portion along the axial direction of the snare device is 1/10-2/5.

22. The snare device according to claim 19, wherein

the stiffness of the third connection segment is the same as the stiffness of the second connection segment; or

the stiffness of the third connection segment is greater than the stiffness of the second connection segment; or

the stiffness of the third connection segment is less than the stiffness of the second connection segment.

23. The snare device according to claim 22, wherein

when the stiffness of the third connection segment is the same as the stiffness of the second connection segment, a ratio range of the stiffness of the first connection segment to the stiffness of the second connection segment is 0.2-0.9; or

when the stiffness of the third connection segment is greater than the stiffness of the second connection segment, a ratio range of the stiffness of the second connection segment, the stiffness of the first connection segment, and the stiffness of the third connection segment is (1.1-3.4):1:(1.4-3.6); or

when the stiffness of the third connection segment is less than the stiffness of the second connection segment, a ratio range of the stiffness of the second connection segment, the stiffness of the first connection segment, and the stiffness of the third connection segment is (1.1-3.5):1:(1-3.2).

24. The snare device according to claim 19, wherein a hardness of the third connection segment is greater than the hardness of the first connection segment.

25. The snare device according to claim 19, wherein the first feature dimension is less than a third feature dimension, wherein

the first feature dimension is a feature dimension of a cross-section of the first connection segment perpendicular to the axial direction of the first connection segment; and

the third feature dimension is a feature dimension of a cross-section of the third connection segment perpendicular to an axial direction of the third connection segment.

26. The snare device according to claim 25, wherein a ratio range of the first feature dimension to the third feature dimension is 0.5-0.95.

27. The snare device according to claim 19, wherein

the first connection segment includes the at least two first connection units, the third connection segment includes at least one third connection unit; and

the first count of strands is greater than a third count of strands, wherein the first count of strands is the count of the first connection units of the first connection segment, and the third count of strands is a count of a third connection unit of the third connection segment.

28. The snare device according to claim 27, wherein when an absolute value of a difference between the first feature dimension and a third feature dimension is less than or equal to a second preset threshold, the first count of strands is greater than the third count of strands, wherein

the first feature dimension is a feature dimension of a cross-section of the first connection segment perpendicular to the axial direction of the first connection segment; and

the third feature dimension is a feature dimension of a cross-section of the third connection segment perpendicular to an axial direction of the third connection segment.

29. The snare device according to claim 28, wherein the second preset threshold is any value between 0mm and 0.1 mm.

30. The snare device according to claim 19, wherein a shape of a third cross-section is a circle, a square, a hexagon, or a combination thereof, and a shape of the first cross-section is a semicircle, a triangle, a rectangle, or a combination thereof, wherein

the first cross-section is a cross-section of the first connection segment perpendicular to the axial direction of the first connection segment; and

the third cross-section is a cross-section of the third connection segment perpendicular to an axial direction of the third connection segment.

31. The snare device according to claim 19, wherein the third connection segment includes a base connection segment and a stiffness reinforcement member, the stiffness reinforcement member being fixed on the base connection segment.

32. The snare device according to claim 1, wherein

the snare portion is a symmetrical structure with an axis along an axial direction of the snare device as a symmetry axis; and/or

the second rigid portion is a symmetrical structure with an axis along the axial direction of the snare device as a symmetry axis.

33. The snare device according to claim 1, wherein the connection portion includes a first traction member and a sheath tube, wherein

the first traction member is disposed in the sheath tube;

the first traction member connects the snare portion and the operation portion; and

the operation portion drives a part or all of the snare portion to extend out of or enter the sheath tube through the first traction member.

34. The snare device according to claim 33, wherein

at least a portion of one of the first rigid portion and the second rigid portion that is closer to the first traction member is an integral structure with the first traction member; and/or

the at least a portion of one of the first rigid portion and the second rigid portion that is closer to the first traction member is fixedly connected to the first traction member.

35. The snare device according to claim 33, wherein the snare portion includes a base portion and an adjustment portion, wherein

the adjustment portion is slidably connected to the base portion;

two ports at a proximal end of the base portion are connected to a distal end of the first traction member;

the adjustment portion includes the first rigid portion and the second rigid portion; and

the operation portion drives a part or all of the base portion and a part or all of the adjustment portion to extend out of or enter the sheath tube.

36. The snare device according to claim 35, wherein the connection portion further includes a second traction member, the second traction member being disposed in the sheath tube, wherein the second traction member and the first traction member are both configured to be movable along an axial direction of the sheath tube, and the second traction member is slidable relative to the first traction member, wherein

the second traction member includes a first pulling cable and a second pulling cable, wherein a port at a distal end of the first pulling cable is connected to a port at a proximal end of the adjustment portion, and a port at a distal end of the second pulling cable is connected to another port at a proximal end of the adjustment portion; and

a sliding movement of the first pulling cable and the second pulling cable along the axial direction of the sheath tube drives the adjustment portion to slide relative to the base portion.

37. The snare device according to claim 33, wherein the operation portion includes an adjustment member, wherein the adjustment member is connected to two ports at a proximal end of the snare portion through the first traction member, and the adjustment member drives the two ports at the proximal end of the snare portion to move synchronously or asynchronously.

38. The snare device according to claim 1, wherein the first rigid portion and/or the second rigid portion are configured with a marker.

FIG. 1

FIG.2

FIG. 3

FIG.4

FIG. 5

EP 4 755 324 A1

**FIG. 6A**

**FIG. 6B**

30

FIG. 7

**FIG. 8A**

**FIG. 8B**

FIG. 8C

FIG. 8D

130 132

131

**FIG. 8E**

130 132

131

**FIG. 8F**

131

131-a

FIG. 9

132

132-1

132-2

131

133

FIG. 10

FIG. 11

FIG. 12A

FIG. 12B

**FIG. 13**

**FIG. 14**

**FIG. 15**

FIG. 16

## INTERNATIONAL SEARCH REPORT

| International application No. |
|---|
| **PCT/CN2024/076187** |

| A. | CLASSIFICATION OF SUBJECT MATTER |
|---|---|

A61B17/3205(2006.01)i; A61B17/32(2006.01)i; A61B18/14(2006.01)i

According to International Patent Classification (IPC) or to both national classification and IPC

| B. | FIELDS SEARCHED |
|---|---|

Minimum documentation searched (classification system followed by classification symbols)

IPC: A61B

Documentation searched other than minimum documentation to the extent that such documents are included in the fields searched

Electronic data base consulted during the international search (name of data base and, where practicable, search terms used)

CNTXT: ENTXTC; ENTXT; OETXT; VEN; CNKI: 杭州安杰思医学科技, 韩春琦, 吴家微, 程永华, 刚度, 刚性, 硬度, 柔, 增强, 加强, 强度, 形态, 形状, 形变, 变形, 贴合, 滑脱, 切割, 环切, 圈套, 抓捕套, 环套, 勒除, 线, 绳, 索, 缆, stiffness, rigid, flex+, ring, sleeve, reinforce, deform, shape, cut, wire, string, cable

| C. | DOCUMENTS CONSIDERED TO BE RELEVANT |
|---|---|

| Category* | Citation of document, with indication, where appropriate, of the relevant passages | Relevant to claim No. |
|---|---|---|
| PX | CN 117281588 A (HANGZHOU AGS MEDTECH CO., LTD.) 26 December 2023 (2023-12-26)<br>claims 1-14, description, paragraphs [0005]-[0080], and figures 1-9 | 1-38 |
| X | CN 211911735 U (HANGZHOU AGS MEDTECH CO., LTD.) 13 November 2020 (2020-11-13)<br>description, paragraphs [0005]-[0034], and figures 1-11 | 1-18, 32-34, 37, 38 |
| A | CN 105813583 A (BOSTON SCIENTIFIC SCIMED, INC.) 27 July 2016 (2016-07-27)<br>entire document | 1-38 |
| A | US 2017049471 A1 (BOSTON SCIENTIFIC SCIMED, INC.) 23 February 2017 (2017-02-23)<br>entire document | 1-38 |
| A | US 2017007279 A1 (SHARMA VIRENDER K.) 12 January 2017 (2017-01-12)<br>entire document | 1-38 |
| A | CN 112386308 A (NATIONAL UNIVERSITY CORPORATION OSAKA UNIVERSITY et al.) 23 February 2021 (2021-02-23)<br>entire document | 1-38 |

☑ Further documents are listed in the continuation of Box C.  ☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **04 June 2024** | **10 June 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

| International application No. |
|---|
| **PCT/CN2024/076187** |

**C.** **DOCUMENTS CONSIDERED TO BE RELEVANT**

| Category* | Citation of document, with indication, where appropriate, of the relevant passages | Relevant to claim No. |
|---|---|---|
| A | CN 101066201 A (ETHICON ENDO-SURGERY INC.) 07 November 2007 (2007-11-07) entire document | 1-38 |
| A | CN 204049839 U (HANGZHOU AGS MEDTECH CO., LTD.) 31 December 2014 (2014-12-31) entire document | 1-38 |
| A | CN 110584749 A (MICRO-TECH (NANJING) CO., LTD.) 20 December 2019 (2019-12-20) entire document | 1-38 |

Form PCT/ISA/210 (second sheet) (July 2022)

**INTERNATIONAL SEARCH REPORT**
Information on patent family members

International application No.

**PCT/CN2024/076187**

| Patent document cited in search report | | | Publication date (day/month/year) | Patent family member(s) | | | Publication date (day/month/year) |
|---|---|---|---|---|---|---|---|
| CN | 117281588 | A | 26 December 2023 | None | | | |
| CN | 211911735 | U | 13 November 2020 | None | | | |
| CN | 105813583 | A | 27 July 2016 | None | | | |
| US | 2017049471 | A1 | 23 February 2017 | US | 10610254 | B2 | 07 April 2020 |
| US | 2017007279 | A1 | 12 January 2017 | None | | | |
| CN | 112386308 | A | 23 February 2021 | None | | | |
| CN | 101066201 | A | 07 November 2007 | CN | 101066201 | B | 08 December 2010 |
| CN | 204049839 | U | 31 December 2014 | None | | | |
| CN | 110584749 | A | 20 December 2019 | None | | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202311219288
- CN 202311346415X